(19) Europäisches Patentamt
European Patent Office
Office européen des brevets

(11) **EP 4 539 733 B1**

(12) **EUROPEAN PATENT SPECIFICATION**

(45) Date of publication and mention
of the grant of the patent:
**03.09.2025 Bulletin 2025/36**

(21) Application number: **23731255.8**

(22) Date of filing: **07.06.2023**

(51) International Patent Classification (IPC):
*A61B 5/021* (2006.01)     *A61B 5/024* (2006.01)
*A61B 5/026* (2006.01)     *A61B 5/029* (2006.01)
*A61B 5/08* (2006.01)      *A61B 5/00* (2006.01)
*A61B 5/02* (2006.01)

(52) Cooperative Patent Classification (CPC):
**A61B 5/02028; A61B 5/021; A61B 5/024;
A61B 5/026; A61B 5/029; A61B 5/0816;
A61B 5/7221; A61B 5/7235**

(86) International application number:
**PCT/EP2023/065177**

(87) International publication number:
**WO 2023/242011 (21.12.2023 Gazette 2023/51)**

(54) **SYSTEMS AND METHODS FOR DETERMINING A FLUID RESPONSIVENESS PARAMETER AND A HEMODYNAMIC PARAMETER**

SYSTEME UND VERFAHREN ZUR BESTIMMUNG EINES FLUIDRESPONSE-PARAMETERS UND EINES HÄMODYNAMISCHEN PARAMETERS

SYSTÈMES ET MÉTHODES POUR DÉTERMINER UN PARAMÈTRE DE RÉPONSE AUX FLUIDES ET UN PARAMÈTRE HÉMODYNAMIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **17.06.2022 EP 22179503**

(43) Date of publication of application:
**23.04.2025 Bulletin 2025/17**

(73) Proprietor: **Koninklijke Philips N.V.
5656 AG Eindhoven (NL)**

(72) Inventors:
• **DERKX, Rene Martinus Maria
5656 AG Eindhoven (NL)**
• **WIJSHOFF, Ralph Wilhelm Christianus Gemma
Rosa
5656 AG Eindhoven (NL)**

(74) Representative: **Philips Intellectual Property &
Standards
High Tech Campus 52
5656 AG Eindhoven (NL)**

(56) References cited:
**US-A1- 2008 033 306     US-A1- 2011 077 532
US-A1- 2020 260 969**

**Description**

FIELD OF THE INVENTION

The invention relates to a system and a method for determining a fluid responsiveness parameter, and a system and a method for determining a hemodynamic parameter. The invention also relates to computer programs corresponding to the systems and methods.

BACKGROUND OF THE INVENTION

US2011077532A1 relates to methods and apparatuses for dynamic estimation of fluid responsiveness.
US2020260969A1 relates to techniques for predicting fluid responsiveness in critically ill patients.
US2008033306A1 relates to an apparatus and method for determining a physiologic parameter of a patient, wherein the parameter is usable to characterize volume responsiveness.
The fluid responsiveness of a patient refers to an increase in cardiac output after administration of fluid. Knowing the fluid responsiveness of a patient can be very important for a clinician in the surgery setting in order to assess the amount of fluid in the patient and to decide whether the patient requires extra fluid intake. In order to quantify the fluid responsiveness of the patient, several fluid responsiveness parameters (FRP) are known. However, not all of them are clinically accepted in all situations. For instance, fluid responsiveness parameters like the pulse pressure variation (PPV) or the less established systolic pressure variation (SPV) are at present only clinically accepted when being assessed in a stable heart situation and a stable respiration situation. These fluid responsiveness parameters can usually only be accurately estimated for patients receiving ventilation with a fixed setting of the ventilation rate and not suffering from heart problems like, for instance, ectopic beats or arrhythmias, since the evaluations of the blood pressure measurements needed therefor become relatively complex and can become inaccurate because of several assumptions that need to be made otherwise.
Apart from being of direct assistance for a clinician in the surgery setting to be provided with a fluid responsiveness parameter for a patient, such as via a display, in order to be able to interpret the provided fluid responsiveness parameter and take clinical measures based thereon, fluid responsiveness parameters can also be used for determining hemodynamic parameters, wherein also these further parameters can be of assistance for a clinician. Again, however, fluid responsiveness parameters can often only be used for accurately determining hemodynamic parameters in stable heart situations. Moreover, many of the hemodynamic parameters that can be determined based on fluid responsiveness parameters are usually only used for ventilated patients, i.e. in stable respiration situations.
Irrespective of whether the fluid responsiveness parameter is used for direct interpretation by a clinician or for indirect interpretation via a hemodynamic parameter determined based on the fluid responsiveness parameter, it is important to know when the fluid responsiveness parameter can be trusted. An assessment of the reliability of an estimated fluid responsiveness parameter can be challenging, particularly when the fluid responsiveness parameter is determined based on a relatively short measurement, such as a measurement covering only a few respiration cycles. However, short measurement durations may be necessary in some situations. For instance, the fluid responsiveness of the patient might need to be estimated during short duration maneuvers like passive leg raise, or in the course of spot checks carried out by a clinician using, for instance, a measurement cuff.
For these reasons an efficient determination of a fluid responsiveness parameter, its reliability and a hemodynamic parameter correlated with the fluid responsiveness parameter is needed.

SUMMARY OF THE INVENTION

It is an object of the invention to allow for an efficient determination of a fluid responsiveness parameter. The further above indicated needs are addressed in that the invention also relates to an efficient determination of the reliability of the determined fluid responsiveness parameter and of a hemodynamic parameter correlated with the determined fluid responsiveness parameter.
The invention is defined in the independent claims. The dependent claims define advantageous embodiments.
In a first aspect, a system for determining a fluid responsiveness parameter for a patient is provided, according to claim 1.
It has been found that the second fluid responsiveness parameter determination signal can carry sufficient information about an influence of the respiration of the patient on the series of blood pulses indicated by the measured blood pulsation signal, while the first fluid responsiveness parameter determination signal can carry sufficient additional information for determining the fluid responsiveness parameter. The fluid responsiveness parameter can therefore be efficiently determined. This is particularly advantageous in situations where the fluid responsiveness parameter needs to be determined fast, i.e. without access to long series of blood pulses, such as for quick spot-checks during surgery. The determination of the fluid responsiveness parameter has also been found to be robust, i.e. showing a good accuracy across various different provided blood pulsations signals, particularly signals acquired for different patients and with different measurement devices.

The first fluid responsiveness parameter determination signal preferably corresponds to the processed signal at a whole spectral range, i.e. all frequencies, up to the respiratory rate, and the second fluid responsiveness parameter determination signal preferably corresponds to the processed signal at the respiratory and all of its harmonics up to the heart rate. The first fluid responsiveness parameter determination signal can particularly comprise a continuous spectrum up to the respiratory rate, while the second fluid responsiveness parameter determination signal can comprise a discrete spectrum consisting of the respiratory rate and its harmonics up to the heart rate.

A correspondence between signals in a spectral range or at particular frequencies like the respiratory rate and its harmonics shall be preferably understood herein as referring to a correspondence, particularly an equality, of respective spectral components of the signals, wherein the spectral components can be determined by a spectral analysis. For instance, a first signal corresponding to a second signal in a spectral range between a frequency f1 and a frequency f2, and additionally at a frequency f3, shall preferably be understood such that the spectrum of the first signal corresponds to the spectrum of the second signal between f1 and f2, and additionally at f3. A frequency shall be preferably understood herein as being equivalent to a rate. A harmonic of a frequency shall be preferably understood herein as referring to an integer multiple of the frequency.

The respiratory rate providing unit, the heart rate providing unit and the blood pulsation signal providing unit can each be a respective receiving unit configured to receive the respective quantity from, for instance, a measurement device and to provide the received quantity. However, these providing units can also be storages in which the previously measured respective quantity has been stored and from which it can be retrieved for providing the same. The providing units can also be or comprise the measurement device which measures the respective quantity.

The respiratory rate and the heart rate can be provided specifically for the patient for whom the fluid responsiveness parameter is to be determined. For instance, the respiratory rate can correspond to a constant ventilation rate with which the patient is ventilated, and the heart rate may correspond to a measured heart or pulse rate at a given time. Even though the heart rate of a patient may change over time, this change may be slow enough to be ignored for a fast fluid responsiveness parameter determination, i.e. when a blood pulsation signal is used that extends over only a relatively short time, such as less than ten, particularly less than five or even only one or two respiratory cycles.

The blood pulsation signal can be, for instance, a tissue pressure signal measured using a pressure sensor in contact with the skin of a patient's limb while a cuff surrounding the limb is inflated to apply increasing pressure to the limb. Such a system and a corresponding measurement method are described, for instance, in EP 3 430 992 A1 and WO 2020/148137 A1. However, the blood pulsation signal can also originate from different measurements. For instance, the blood pulsation signal can be measured in the pressure of an air-filled cuff which is surrounding a limb and inflated to apply increasing pressure to the limb or in a (photo-)plethysmography measurement (PPG) or via a displacement measurement using an inertial measurement unit (IMU).

In the case of a tissue pressure (TP) measurement, an oscillatory component of the measured blood pulsation signal may be named "TPac". The oscillatory component of the blood pulsation signal could also be referred to as an AC component, and could be determined as the difference between the measured blood pulsation signal and its DC component, wherein the DC component of the measured blood pulsation signal can refer to an average, for instance.

The processed signal determining unit can be configured to determine the processed signal based on the blood pulsation signal by first determining an oscillatory component of the blood pulsation signal and then determining the processed signal based on the oscillatory component. For instance, the processed signal can correspond to an enveloping signal, or an envelope in short, of the oscillatory component. The processed signal, particularly the envelope, can be determined by applying a filter, particularly a non-linear filter, to the oscillatory component. An exemplary filter that can be used for this purpose is given by the following equation:

$$y[i] = \begin{cases} y[i-1] + (x[i] - y[i-1]) \cdot (1 - \beta_{up}), & x[i] \geq y[i-1] \\ y[i-1] \cdot \beta_{down}, & x[i] < y[i-1] \end{cases}, \quad (1)$$

where $x[i]$ refers to the input signal, i.e. the oscillatory component of the blood pulsation signal that can particularly be the signal TPac in the case of a tissue pressure measurement, with i being the discrete sample index, and y[i] refers to the computed, i.e. filtered, output signal, and where

$$\beta_{up} = e^{\frac{-1}{Tup \cdot Fs}} \quad (2)$$

and

$$\beta_{down} = e^{\frac{-1}{T_{down} \cdot F_s}} \qquad\qquad (3)$$

are filter parameters computed from time constants $T_{up}$ and $T_{down}$ characterizing a smoothing function of the filter, wherein the constant $F_s$ corresponding to a sampling rate at which the input signal is acquired. For determining the processed signal, the filter parameters can be chosen as $T_{up}= T_{pulse}/2$ and $T_{down}= T_{pulse} \cdot 2$, where $T_{pulse} = 60 / PR$, with PR corresponding to a measured pulse rate provided as a heart rate by the heart rate providing unit, and with $T_{up}, T_{down}, T_{pulse}$ being measured in seconds and PR being measured in beats, or pulses, per minute.

While the processed signal determining unit can be configured to obtain the processed signal by applying the filter to the oscillatory component of the blood pulsation signal, the fluid responsiveness parameter determining unit may be configured to determine a filtered fluid responsiveness parameter determination signal based on the second fluid responsiveness parameter determination signal and a further application of a filter, and to determine the fluid responsiveness parameter based on the first fluid responsiveness parameter determination signal and the filtered fluid responsiveness parameter determination signal.

Hence, a filter can be used to obtain the processed signal, wherein the same or a further filter can be applied when using the second fluid responsiveness parameter determination signal to determine the filtered fluid responsiveness parameter determination signal, but not necessarily to further process the first fluid responsiveness parameter determination signal. In other words, starting from the blood pulsation signal, a filter may only need to be applied once in order to arrive at the first fluid responsiveness parameter determination signal, whereas the same filter may be applied twice, or two different filters may be applied to arrive at the filtered fluid responsiveness parameter determination signal.

For instance, applying a filter having an averaging or smoothing effect to the first fluid responsiveness parameter determination signal may not be needed, since the first fluid responsiveness parameter determination signal, which corresponds to the processed signal in a spectral range up to the respiratory rate and may therefore only comprise relatively low frequencies, may not need any more averaging or smoothing in order to serve as a basis for the fluid responsiveness parameter determination. This is particularly the case if the filter already applied to obtain the processed signal, based on which the first fluid responsiveness parameter determination signal is determined, also has an averaging or smoothing effect.

Preferably, the filter applied to determine the filtered fluid responsiveness parameter determination signal based on the second fluid responsiveness parameter determination signal is again described by above equations (1) to (3), wherein the input $x[i]$ now corresponds to the second fluid responsiveness parameter determination signal or a signal comprising the second fluid responsiveness parameter determination signal, and wherein now the filter parameters are chosen as $T_{up} = T_{resp}/4$ and $T_{down}= T_{resp} \cdot 4$, with $T_{resp}=60 / VR$ and VR referring to the ventilation rate measured in respirations per minute.

Generally, applying appropriate filters to signals like the blood pulsation signal, its oscillatory component or any signal derived therefrom, such as the second fluid responsiveness parameter determination signal, can be an efficient way of emphasizing the respective signal properties of interest. In signals comprising several pulses or oscillations as discussed herein, for instance, it may be of interest how the pulses or oscillations relate to each other, or develop. Hence, filters may be preferred that result in a respective filtered signal indicating how the pulses or oscillations in the input signal relate to each other, or develop. For instance, filters may be preferred that result in envelopes, or signals indicative of envelopes, of the respective input signal. The filters may be chosen to depend on the provided respiratory rate and/or heart rate. Also, filters can be designed that exploit a fiducial point detection of maxima and apply interpolation between the detected values.

The fluid responsiveness parameter determining unit can be configured to determine the fluid responsiveness parameter based on characteristic values of the first fluid responsiveness parameter determination signal and the filtered fluid responsiveness parameter determination signal. This can further increase the efficiency of the fluid responsiveness parameter determination, since not the whole signals need to be analyzed. The characteristic values can particularly refer to global or local maxima. For instance, a characteristic position may be identified in one of the first fluid responsiveness parameter determination signal and the filtered fluid responsiveness parameter determination signal, wherein then the value of the respective one of the signals at this position and a value of the other of the first fluid responsiveness parameter determination signal and the filtered fluid responsiveness parameter determination signal at the same position may be identified, and the two identified values may be used for determining the fluid responsiveness parameter. A position in a signal may correspond to a point in time during measurement of the respective signal. If the blood pulsation signal is a tissue pressure signal measured using a pressure sensor in contact with the skin of a patient's limb while a cuff surrounding the limb is inflated to apply increasing pressure to the limb, for instance, a point in time during measurement may also correspond to a corresponding pressure being applied by the cuff.

The processed signal determining unit can be configured to determine a first processed signal obtained by applying a filter to an oscillatory component of the blood pulsation signal and a second processed signal obtained by inverting the oscillatory component and applying the filter to the inverted oscillatory component, wherein the fluid responsiveness parameter determining unit can be configured to determine the fluid responsiveness parameter by a') identifying a first fluid responsiveness parameter determination signal for each of the first processed signal and the second processed signal and combining the first fluid responsiveness parameter determination signals to a combined first fluid responsiveness parameter determination signal, identifying a second fluid responsiveness parameter determination signal for the first processed signal, determining a filtered fluid responsiveness parameter determination signal by applying a filter to the second fluid responsiveness parameter determination signal identified for the first processed signal, and b') determining the fluid responsiveness parameter based on the combined first fluid responsiveness parameter determination signal and the filtered fluid responsiveness parameter determination signal. In this case, the determined fluid responsiveness parameter can particularly be a systolic pressure variation (SPV).

In order to determine the systolic pressure variation, the system may further comprise a blood pressure characteristic providing unit for providing a blood pressure characteristic, wherein the fluid responsiveness parameter determining unit is configured to determine the fluid responsiveness parameter based further on the blood pressure characteristic. The blood pressure characteristic may correspond to or be derived from a systolic arterial pressure (SAP) and/or a diastolic arterial pressure (DAP). The systolic pressure variation may however also be determined without reference to any blood pressure characteristic like SAP or DAP.

Also the blood pressure characteristic providing unit can be a receiving unit configured to receive the blood pressure characteristic from, for instance, a measurement device and to provide the received blood pressure characteristic, or a storage in which the previously measured blood pressure characteristic has been stored and from which it can be retrieved for providing the same, or it can be or comprise the measurement device which measures the blood pressure characteristic.

Preferably, the systolic pressure variation is determined in accordance with the following equation:

$$SPV = 2 \cdot \frac{\max\limits_{k-\frac{N}{2} \le n \le k+\frac{N}{2}}(\text{env\_ripple\_up}[n])}{\text{baseline\_sum}[k]} \cdot \frac{\text{SAP} - \text{DAP}}{\text{SAP}} , \quad (4)$$

wherein baseline_sum corresponds to the sum of the two first fluid responsiveness parameter determination signals, env_ripple_up corresponds to the filtered fluid responsiveness parameter determination signal determined for the second fluid responsiveness parameter determination signal, the index $k$ corresponds to the maximum peak in the signal baseline_sum and $N$ indicates a sampled signal length, such as in terms of a number of signal sampling points. As indicated above, Eq. (4) could alternatively be used also without the factor (SAP - DAP)/SAP.

The processed signal determining unit can also be configured to determine a first processed signal obtained by applying a filter to an oscillatory component of the blood pulsation signal and a second processed signal obtained by inverting the oscillatory component and applying the filter to the inverted oscillatory component, wherein the fluid responsiveness parameter determining unit may be configured to determine the fluid responsiveness parameter by a") identifying a first fluid responsiveness parameter determination signal for each of the processed signals and combining them to a combined first fluid responsiveness parameter determination signal, identifying a second fluid responsiveness parameter determination signal for each of the processed signals and combining them to a combined second fluid responsiveness parameter determination signal, and determining a filtered combined second fluid responsiveness parameter determination signal by further applying a filter to the combined second fluid responsiveness parameter determination signal, and b") determining the fluid responsiveness parameter based on the combined first fluid responsiveness parameter determination signal and the filtered combined second fluid responsiveness parameter determination signal. In this case, the fluid responsiveness parameter can particularly be a pulse pressure variation (PPV).

Combining the two second fluid responsiveness parameter determination signals, i.e. the second fluid responsiveness parameter determination signal determined for the first processed signal and the second fluid responsiveness parameter determination signal determined for the second processed signal, can particularly refer to an addition of the two signals. Hence, the combined second fluid responsiveness parameter determination signal can particularly refer to a sum of the second fluid responsiveness parameter determination signal determined for the first processed signal and the second fluid responsiveness parameter determination signal determined for the second processed signal. In case a non-linear filter is used, which may be preferred, it can be advantageous to first determine the combined second fluid responsiveness parameter determination signal and only then, i.e. afterwards, apply the filter to arrive at the filtered combined second fluid responsiveness parameter determination signal, i.e. as opposed to first applying the filter and then combining the filtered signals.

Preferably, only the combined first fluid responsiveness parameter determination signal and the filtered combined second fluid responsiveness parameter determination signal are used for determining PPV. Hence no further signals or data need to be considered, which can make the fluid responsiveness parameter yet more efficient. For instance, the pulse pressure variation may be determined in accordance with the following equation:

$$PPV = 2 \cdot \frac{\max\limits_{k-\frac{N}{2} \le n \le k+\frac{N}{2}}(\text{env\_ripple\_sum}[n])}{\text{baseline\_sum}[k]} \, , \qquad (5)$$

wherein baseline_sum corresponds to the sum of the two first fluid responsiveness parameter determination signals, env_ripplesum corresponds to the filtered sum of the two second fluid responsiveness parameter determination signals, the index $k$ corresponds to the maximum peak in the signal baseline_sum and $N$ indicates a sampled signal length, such as in terms of a number of signal sampling points.

The determination of the processed signal based on the blood pulsation signal by the processed signal determining unit may involve a regularization of detected blood pulses in the blood pulsation signal that are due to ectopic beats. This can make the assumption of a constant heart rate more applicable even in the case of heart problems or signal artefacts caused by other reasons. The regularization can refer, for instance, to inserting artificial pulses in the series of blood pulses at positions where a pulse is expected, but not present or only with an amplitude below a predetermined threshold.

Also, a system for determining a hemodynamic parameter correlated with a fluid responsiveness parameter is presented according to claim 8.

It has been found that the reliability determination signal carries important information about the reliability of the determined fluid responsiveness parameter. Together with the second fluid responsiveness parameter determination signal, it therefore allows for an efficient determination of the reliability of the determined fluid responsiveness parameter determination signal. In applications where the fluid responsiveness parameter is determined based on only relatively short series of blood pulses, knowing the reliability of the determined fluid responsiveness parameter is particularly advantageous, since the determination is then particularly sensitive to irregularities in the series of blood pulses. Such irregularities can be caused by ectopic beats or correspond to other signal artefacts. For instance, the reliability of a determined fluid responsiveness parameter can be measured in terms of a quality index taking values between 0 and 100 %. Different quality indices may be employed for different fluid responsiveness parameters.

Preferably, the quality index

$$\text{SPV QI} = 1 - \frac{\sum_{n=k-N/2}^{k+N/2}|\text{ripple\_up}[n] - \text{ripple\_up2}[n]|}{\sum_{n=k-\frac{N}{2}}^{k+\frac{N}{2}}|\text{ripple\_up}[n] + \text{ripple\_up2}[n]|} \qquad (6)$$

is used for SPV, and the quality index

$$\text{PPV QI} = 1 - \frac{\sum_{n=k-\frac{N}{2}}^{k+\frac{N}{2}}|\,\text{ripple\_sum}[n] - \text{ripple\_sum2}[n]\,|}{\sum_{n=k-\frac{N}{2}}^{k+\frac{N}{2}}|\,\text{ripple\_sum}[n] + \text{ripple\_sum2}[n]\,|} \qquad (7)$$

is used for PPV, wherein ripple_up corresponds to the second fluid responsiveness parameter determination signal determined for a first processed signal indicative of an upper envelope of the blood pulsation signal, ripple_up2 corresponds to the reliability determination signal determined for this processed signal, ripple_sum corresponds to a sum of the second fluid responsiveness parameter determination signal determined for this processed signal and a second fluid responsiveness parameter determination signal determined for a processed signal indicative of a lower envelope of the blood pulsation signal, ripple_sum2 corresponds to a sum of the reliability determination signal determined for the processed signal indicative of the upper envelope of the blood pulsation signal and a reliability determination signal determined for the processed signal indicative of the lower envelope of the blood pulsation signal, and $k$ and $N$ are defined as for determining SPV and PPV, respectively.

Since the fluid responsiveness parameter can be efficiently determined with the above-described system for determining a fluid responsiveness parameter, the hemodynamic parameter correlated with the fluid responsiveness parameter can be efficiently determined as well.

The hemodynamic parameter being correlated with the fluid responsiveness parameter shall be preferably understood such that a model can be found expressing the former in terms of the latter, wherein the model can refer to a function, for instance. The hemodynamic parameter can particularly be a stroke volume (SV). The combined model can particularly involve a weighting of the first model and the second model, wherein the weights depend on the reliability of the fluid responsiveness parameter such that, with increasing reliability of the fluid responsiveness parameter, the weight of the first model increases relative to the weight of the second model. The weight of the first model and the weight of the second model preferably sum up to 1.

Another aspect relates to a computer-implemented method for determining a fluid responsiveness parameter for a patient, according to claim 9.

A further aspect concerns a computer-implemented method for determining a hemodynamic parameter correlated with a fluid responsiveness parameter, according to claim 10.

According to further aspects, computer programs corresponding to the above-described systems and methods are provided.

Thus, a computer program for determining a fluid responsiveness parameter for a patient is provided, according to claim 11.

Furthermore, a computer program for determining a hemodynamic parameter correlated with a fluid responsiveness parameter is provided, according to claim 12.

It shall be understood that the systems of claims 1 and 8, the methods of claims 9, and 10 and the computer programs of claims 11 and 12 have similar and/or identical preferred embodiments, particularly as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a system for determining a fluid responsiveness parameter for a patient,
Fig. 2 shows schematically and exemplarily a blood pulsation signal as well as a DC and an AC component of the blood pulsation signal,
Fig. 3 shows schematically and exemplarily the oscillatory component of the blood pulsation signal and a first and a second processed signal determined based on the oscillatory component,
Fig. 4A shows schematically and exemplarily a first fluid responsiveness parameter determination signal, a second fluid responsiveness parameter determination signal and a reliability determination signal determined based on the first processed signal,
Fig. 4B shows schematically and exemplarily a first fluid responsiveness parameter determination signal, a second fluid responsiveness parameter determination signal and a reliability determination signal determined based on the second processed signal,
Fig. 5 shows schematically and exemplarily a system for determining a reliability of a fluid responsiveness parameter determined by a system as shown in Fig. 1,
Fig. 6 shows schematically and exemplarily several signals derived from the first fluid responsiveness parameter determination signal, the second fluid responsiveness determination signal and the reliability determination signal shown in Figs. 4A and 4B,
Fig. 7A shows schematically and exemplarily a conventional definition of systolic pressure variation,
Fig. 7B shows schematically and exemplarily a conventional definition of pulse pressure variation,
Fig. 8A shows schematically and exemplarily a blood pulsation signal comprising a pressure pulse due to an ectopic beat, as well as an average and an oscillatory component of this blood pulsation signal,
Fig. 8B shows schematically and exemplarily the oscillatory component of the blood pulsation signal shown in Fig. 8A, and a first and a second processed signal determined based thereon,
Fig. 8C shows schematically and exemplarily a first fluid responsiveness parameter determination signal, a second fluid responsiveness parameter determination signal and a reliability determination signal determined based on the first processed signal shown in Fig. 8B,
Fig. 8D shows schematically and exemplarily a first fluid responsiveness parameter determination signal, a second fluid responsiveness parameter determination signal and a reliability determination signal determined based on the second processed signal shown in Fig. 8B,
Fig. 8E shows schematically and exemplarily several signals derived from the first fluid responsiveness parameter determination signal, the second fluid responsiveness determination signal and the reliability determination signal shown in Figs. 8C and 8D,
Fig. 9 shows schematically and exemplarily a system for determining a hemodynamic parameter,
Fig. 10 shows schematically and exemplarily a method for determining a fluid responsiveness parameter,

Fig. 11 shows schematically and exemplarily a method for determining a reliability of a fluid responsiveness parameter determined by the method shown in Fig. 10,

Fig. 12 shows schematically and exemplarily a method for determining a hemodynamic parameter,

Fig. 13 shows schematically and exemplarily specific details of determining the signals shown in Figs. 4A, 4B, 8C and 8D,

Fig. 14A shows schematically and exemplarily specific details of determining systolic pressure variation and its reliability by using some of the signals determined as shown in Fig. 13,

Fig. 14B shows schematically and exemplarily specific details of determining pulse pressure variation and its reliability by using all of the signals determined as shown in Fig. 13, and

Fig. 15 shows schematically and exemplarily specific details of determining a stroke volume SV.

DETAILED DESCRIPTION OF EMBODIMENTS

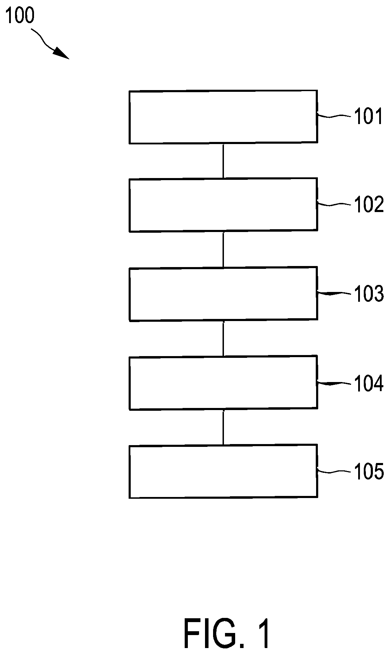

Fig. 1 shows schematically and exemplarily the system 100 for determining a fluid responsiveness parameter (FRP) for a patient. The system 100 comprises a respiratory rate providing unit 101 that is configured to provide a respiratory rate, and a heart rate providing unit 102 that is configured to provide a heart rate. The respiratory rate providing unit 101 and the heart rate providing unit 102 can be part of or correspond to a single providing unit providing both the respiratory rate and the heart rate. The provided respiratory rate and/or heart rate can have values that are assumed to be known a priori. For instance, if the patient is under intensive care, he or she may be externally ventilated, and a pulse of the patient may be continuously measured. Then, the provided respiratory rate may correspond to the ventilation rate, which will typically be held constant over extended periods of time, and the provided heart rate may correspond to a measured pulse rate at a given time. The heart or pulse rate may also be assumed to be constant, particularly for relatively short measurement times used for acquiring any signals needed for the determination of the fluid responsiveness parameter. The respiratory rate and the heart rate may be provided by the providing units based on a previously received input, wherein the input may be provided automatically based on, for instance, control parameters of a ventilation system and pulse measurement values, or may be provided manually, such as via a user interface of the system 100.

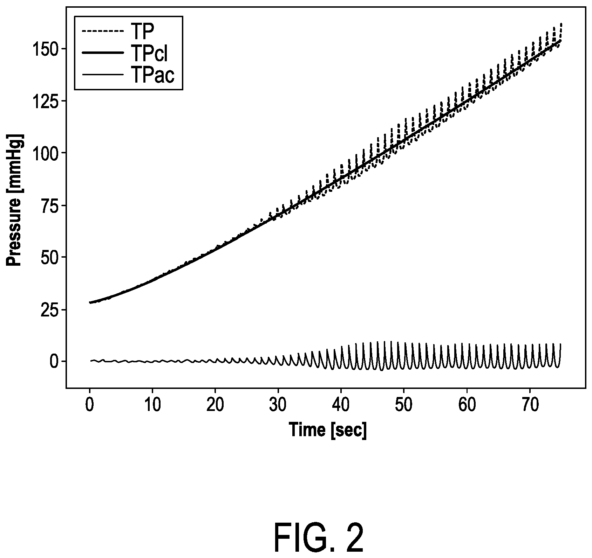

The system 100 further comprises a blood pulsation signal providing unit 103 that is configured to provide a blood pulsation signal, wherein the blood pulsation signal is a measured signal indicative of a series of blood pulses of the patient. For instance, the blood pulsation signal can be provided based on a measurement using a dedicated cuff system as described in EP 3 430 992 A1, and correspond, for instance, to a tissue pressure signal TP acquired as described in WO 2020/148137 A1. Fig. 2 shows an example of a measured tissue pressure signal TP to which the subsequently described embodiments relate. In other embodiments, other types of blood pulsation signals may be used. As seen in Fig. 2, the tissue pressure signal TP comprises a plurality of peaks, each of which is indicative of one of a series of blood pulses of the patient. The fact that the signal TP shown in Fig. 2 is generally increasing over time is due to the measurement method, according to which a pressure applied by the cuff system from outside to the patient's skin is continuously increased over a measurement time in this case.

The system 100 further comprises a processed signal determining unit 104 that is configured to determine two processed signals, referred to herein as env_up and env_down, based on the blood pulsation signal TP, wherein these processed signals are indicative of a respective envelope of the blood pulsation signal TP. More specifically, the signal env_up is determined such that it is indicative of an upper envelope of the blood pulsation signal TP, and the signal env_down is determined such that it is indicative of a lower envelope of the blood pulsation signal TP. For instance, based on the measured blood pulsation signal corresponding to the tissue pressure TP, an oscillatory component TPac thereof may be determined, such as by applying a highpass filter to the blood pulsation signal TP. In this way, the blood pulsation signal TP can be decomposed into a DC component and an AC component, wherein the DC component corresponds to a clamping pressure TPcl applied from outside to the patient's skin during measurement and could be regarded as being indicative of an average of the blood pulsation signal TP, and the AC component corresponds to the signal TPac. This decomposition is illustrated exemplarily in Fig. 2.

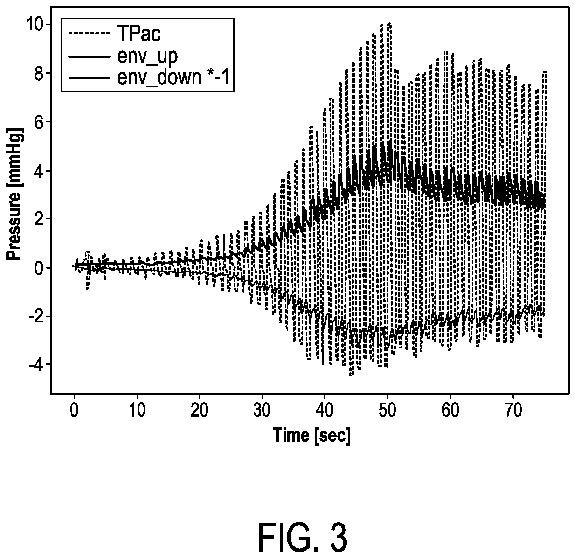

In Fig. 3, the signal component TPac is shown without the original signal TP and its DC component TPcl. Instead, the processed signal env_up and the inverted version -env_down of the processed signal env_down, which itself is positive, are additionally shown in Fig. 3. As will be appreciated from Fig. 3, the signal env_up, particularly its DC component, resembles the upper envelope of the oscillatory component TPac of the blood pulsation signal TP, and the signal env_down, particularly its DC component, resembles, up to the inversion, the lower envelope of TPac. The signals env_up and env_down can therefore also be referred to as "envelope" signals.

The system 100 further includes a fluid responsiveness parameter determining unit 105 that is configured to determine the fluid responsiveness parameter FRP by a) identifying two first fluid responsiveness parameter determination signals, which correspond to the "baseline" signals baseline_up and baseline_down in the illustrated embodiments, based on the processed signals env_up and env_down, wherein the first fluid responsiveness parameter determination signals baseline_up and baseline_down correspond to the processed signals env_up and env_down in a spectral range up to the respiratory rate, i.e. the respiratory rate previously provided. In particular, the signal baseline_up corresponds to the signal env_up in the spectral range up to the respiratory rate, and the signal baseline_down corresponds to the signal env_down in the spectral range up to the respiratory rate. Furthermore, the fluid responsiveness parameter determining unit 105 is configured to identify two second fluid responsiveness parameter determination signals, which correspond to the "ripple" signals ripple_up and ripple_down in the illustrated embodiments, based on the processed signals env_up and env_down, wherein the second fluid responsiveness parameter determination signals ripple_up and ripple_down correspond to the processed signals env_up and env_down at the respiratory rate and any of its harmonics up to the heart rate, i.e. up to the previously provided heart rate. In particular, the signal ripple_up corresponds to the signal env_up at the respiratory rate and any of its harmonics up to the heart rate, and the signal ripple_down corresponds to the processed signal env_down at the respiratory rate and any of its harmonics up to the heart rate. Hence, for instance, the first fluid responsiveness parameter determination signals baseline_up and baseline_down can comprise a continuous spectrum of the respective envelope signal up to the respiratory rate, while the second fluid responsiveness parameter determination signals ripple_up and ripple_down can comprise a discrete spectrum consisting of signal values corresponding to those of the respective envelope signal at the respiratory rate and its harmonics up to the heart rate.

The fluid responsiveness parameter determining unit 105 is configured to determine the fluid responsiveness parameter FRP based on the identified first fluid responsiveness parameter determination signals baseline_up and baseline_down and the identified second fluid responsiveness parameter determination signals ripple_up and ripple_down. As will be exemplarily described further below, for some fluid responsiveness parameters, not all of these fluid responsiveness parameter determination signals may be needed, however.

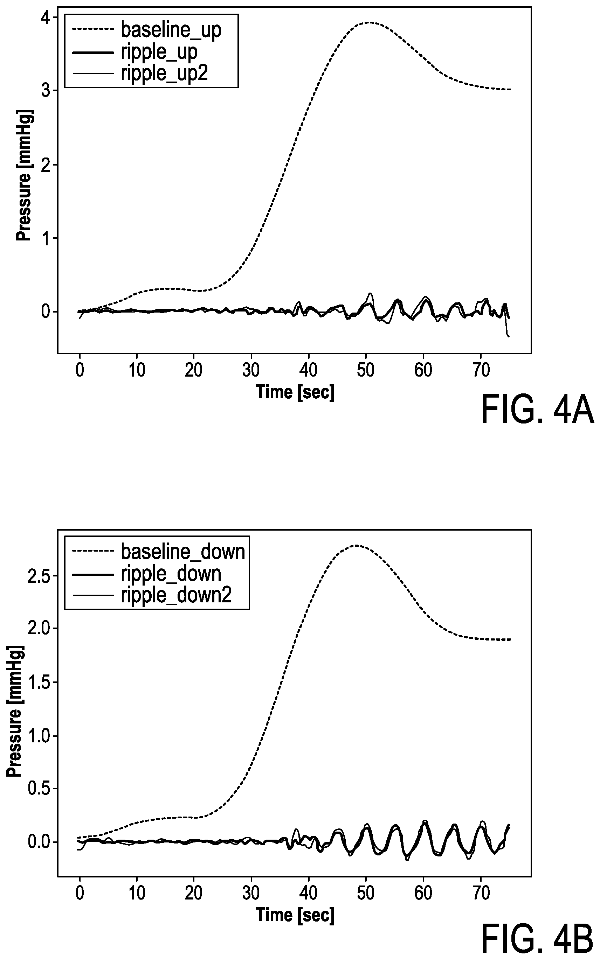

Fig. 4A and Fig. 4B illustrate schematically and exemplarily the first and second fluid responsiveness parameter determination signals identified based on the processed signals env_up and env_down, wherein Fig. 4A shows the signals baseline_up and ripple_up identified based on the signal env_up, and Fig. 4B shows the signals baseline_down and ripple_down identified based on the signal env_down. Moreover, Figs. 4A and Fig. 4B show further signals ripple_up2 and ripple_down2, respectively.

The signals ripple_up2 and ripple_down2 are examples of reliability determination signals identified based on the same processed signals env_up and env_down based on which the first and second fluid responsiveness parameter determination signals are identified, in order to determine a reliability of the fluid responsiveness parameter determined by the system 100. For instance, the system 100 can comprise a reliability determining unit that is configured to determine a reliability Q of the fluid responsiveness parameter FRP determined by the fluid responsiveness parameter determining unit 105 by a) identifying the reliability determination signals ripple_up2 and ripple_down2 based on the processed signals env_up and env_down, particularly by identifying the signal ripple_up2 based on the signal env_up and the signal ripple_down2 based on the signal env_down, wherein the reliability determination signals ripple_up2 and ripple_down2 correspond to the respective processed signal env_up, env_down in a spectral range between the respiratory rate and the heart rate, and b) determining the reliability Q of the determined fluid responsiveness parameter FRP based on the second fluid responsiveness parameter determination signals ripple_up and ripple_down and the reliability determination signals ripple_up2 and ripple_down2. While the second fluid responsiveness parameter determination signals ripple_up and ripple_down can be regarded as indicating the respiration modulation, i.e. the typically ripple-like modulation of the pulse heights in the measured signal TP, the reliability determination signals can be regarded as being a further kind of ripple-like signal derived from the measured signal TP, and could therefore be regarded as further "ripple" signals. For instance, the reliability determination signals ripple_up2 and ripple_down2 can comprise a continuous spectrum of the respective envelope signal between the respiratory rate and the heart rate.

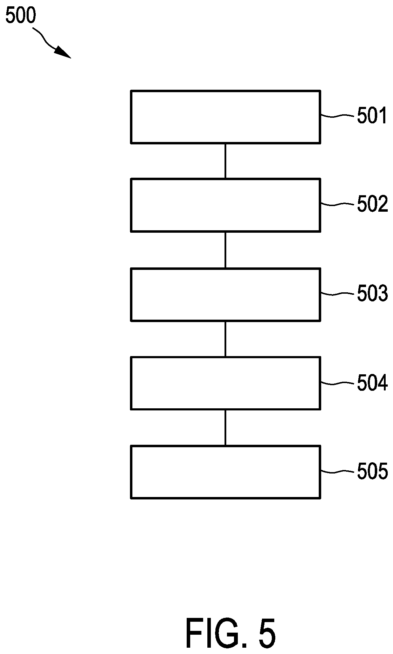

If the system 100 comprises the reliability determining unit, it could also, i.e. additionally, be regarded as a system for determining a reliability of the fluid responsiveness parameter FRP. It can be preferred that the reliability determining unit corresponds to the fluid responsiveness parameter determining unit 105, wherein, in particular, the system 100 can comprise a joint unit configured to perform both the functions described above with respect to the fluid responsiveness parameter determining unit 105 and the functions described above with respect to the reliability determining unit. On the other hand, for a system to determine the reliability of a given fluid responsiveness parameter, it is not necessary that the system itself also determines the fluid responsiveness parameter. Hence, also a system separate from the system 100 can be provided for determining a reliability of the fluid responsiveness parameter like the one determined by the system 100. Such a system is illustrated schematically and exemplarily in Fig. 5.

Fig. 5 shows a system 500 for determining a reliability of a fluid responsiveness parameter FRP determined by the system 100. The system 500 comprises a respiratory rate providing unit 501 that is configured to provide the respiratory rate based on which the fluid responsiveness parameter FRP has been determined. For instance, the respiratory rate providing unit 501 can correspond to the respiratory rate providing unit 101 or receive the respiratory rate from it directly or indirectly after being stored. The system 500 further comprises a heart rate providing unit 502 that is configured to provide the heart rate based on which the fluid responsiveness parameter FRP has been determined. For instance, the heart rate providing unit 502 can correspond to the heart rate providing unit 102 or receive the heart rate from it directly or indirectly after being stored.

The system 500 further comprises a processed signal providing unit 503 that is configured to provide the processed signals env_up and env_down based on which the fluid responsiveness parameter FRP has been determined. The processed signal providing unit 503 can, for instance, receive the processed signals env_up and env_down from the processed signal determining unit 104 after they have been determined. Similarly, the system 500 comprises a second fluid responsiveness parameter determination signal providing unit 504 that is configured to provide the second fluid responsiveness parameter determination signals ripple_up and ripple_down of, i.e. corresponding to, the processed signals env_up and env down, wherein the second fluid responsiveness parameter determination signal providing unit 504 can, for instance, receive the signals ripple_up and ripple _down from the fluid responsiveness parameter determining unit 105 directly or indirectly after being stored.

Moreover, the system 500 comprises a reliability determining unit 505 that is configured to determine the reliability Q of the determined fluid responsiveness parameter FRP by a) identifying the reliability determination signals ripple_up2 and ripple_down2 based on the processed signals env_up and env_down, particularly by identifying the signal ripple_up2 based on the signal env_up and the signal ripple _down2 based on the signal env down, wherein the reliability determination signals ripple_up2 and ripple_down2 correspond to the respective processed signal env_up, env_down in a spectral range between the respiratory rate and the heart rate, and b) determining the reliability Q of the determined fluid responsiveness parameter FRP based on the second fluid responsiveness parameter determination signals ripple_up and ripple_down and the reliability determination signals ripple_up2 and ripple_down2. As will become apparent further below, for determining the reliability of some fluid responsiveness parameters, not all of the reliability determination signals may be needed.

As can be seen in Fig. 4A, the signal ripple_up has less structure than ripple_up2, which is because ripple_up2 has more spectral content, in fact in this case all spectral content between the respiratory rate and the heart rate, whereas ripple_up only focuses on the respiratory rate and its harmonics up to the heart rate. As can be seen in Fig. 4B, for the same reason, only applied to env_down now, ripple_down has less structure than ripple_down2.

In the illustrated embodiments, the processed signal determining unit 104 has obtained the processed signals env_up and env_down by applying a filter to the oscillatory component TPac of the blood pulsation signal TP, wherein the fluid responsiveness parameter determining unit 105 is configured to determine a filtered fluid responsiveness parameter determination signal env_ripple_up based on the second fluid responsiveness parameter determination signal ripple_up, and a further filtered fluid responsiveness parameter determination signal env_ripple_sum based on the second fluid responsiveness parameter determination signals ripple_up and ripple_down. While both of the filtered fluid responsiveness parameter determination signals env_ripple_up and env_ripplesum are determined based on a further application of a filter, the signal env_ripple_up is determined by applying the filter to the second fluid responsiveness parameter determination signal ripple_up itself, such that the signal env_ripple_up could also be referred to as a filtered second fluid responsiveness parameter determination signal, whereas, for determining the signal env_ripple_sum, the filter is not applied to any of the second fluid responsiveness parameter determination signals ripple_up and ripple_down themselves, but a signal derived from them, specifically their sum, as described further below. The fluid responsiveness parameter determining unit 105 is configured to determine the fluid responsiveness parameter FRP based on the first fluid responsiveness parameter determination signals baseline_up and baseline_down and the filtered fluid responsiveness parameter determination signals env_ripple_up and env _ripple_sum.

The filter applied to the oscillatory component TPac to obtain the processed signals env_up and env_down and the filter applied for determining the filtered fluid responsiveness parameter determination signals env_ripple_up and env_ripple_sum can be the same or different from each other. The filter applied to the signal TPac to obtain the signals env_up and env_down can preferably be described according to the following equation:

$$y[i] = \begin{cases} y[i-1] + (x[i] - y[i-1]) \cdot (1 - \beta_{up}), \ x[i] \geq y[i-1] \\ y[i-1] \cdot \beta_{down}, \ x[i] < y[i-1] \end{cases}, \qquad (8)$$

where $x[i]$ refers to the input signal, i.e. in this case the signal TPac, with $i$ being the discrete sample index, and y[i] refers to the computed, i.e. filtered, output signal, and where

$$\beta_{up} = e^{\frac{-1}{Tup \cdot Fs}} \qquad (9)$$

and

$$\beta_{down} = e^{\frac{-1}{Tdown \cdot Fs}} \qquad (10)$$

are filter parameters computed from time constants $T_{up}$ and $T_{down}$ characterizing a smoothing function of the filter, wherein the constant $F_s$ corresponds to a sampling rate at which the input signal is acquired. While env_up can be obtained by directly applying this filter to TPac, env_down is obtained by applying the filter to TPac only after multiplying TPac by a factor of -1, i.e. changing the sign of TPac.

The equation used above to describe the filter applied to obtain the signals env_up and env_down from TPac can be regarded as a recursive averaging equation. The filter, which can be implemented by this equation, can be regarded as an asymmetric filter, since it has an upper prevalence, meaning that the averaging is more directed to positive values. Moreover, the filter is nonlinear. Since the signals env_up and env_down resulting from the filtering can correspond to or resemble envelopes of the input signal TPac, the processed signal determining unit 104 can be regarded as comprising an "envelope detector", wherein this envelope detector may be a part of the processed signal determining unit 104 implementing the above recursive averaging equation for the filtering process. Exemplary choices for the time constants used when the filter is applied to the oscillatory component TPac to obtain the processed signals env_up and env_down are $T_{up} = T_{pulse}/2$ and $T_{down} = T_{pulse} \cdot 2$, where $T_{pulse} = 60 / PR$, with PR corresponding to a measured pulse rate provided as a heart rate by the heart rate providing unit 102, $T_{up}$, $T_{down}$, $T_{pulse}$ being measured in seconds and PR being measured in beats, or pulses, per minute.

The first and second fluid responsiveness parameter determination signals baseline_up, baseline_down, ripple_up and ripple_down, which may also be simply referred to as "baselines" and "ripples", can be identified by the fluid responsiveness parameter determining unit 105 based on the processed signals env_up and env_down, which could also be simply referred to as "envelopes", by a spectral analysis. It could also be said that the baselines and ripples are "extracted" from the envelopes, i.e., for instance, by means of the spectral analysis. In particular, a ventilation rate VR, which may have a value of twelve breaths per minute, for instance, and a pulse rate PR, which may have a value of fifty-two beats per minute, for instance, both of which may be provided by the respective providing units 101 and 102, can be converted into corresponding fundamental harmonic frequencies F0_vr and F0_pr, respectively, wherein these fundamental harmonic frequencies can be used to extract the respective parts of the spectrum corresponding to the signals env_up and env_down. Exemplary values of the ventilation rate and the pulse rate are VR=12 when measured in breaths per minute and PR=52 when measured in beats per minute, wherein the values of the respective fundamental harmonic frequencies would then be given by F0_vr=VR/60=1/5 and F0_pr=PR/60=13/15 when measured in respirations and beats per second, respectively. As indicated already, the extraction of the respective parts of the spectrum corresponding to the signals env_up and env_down, as necessary for the first and second fluid responsiveness parameter determination signals baseline_up, baseline_down, ripple_up and ripple_down, may also be regarded as corresponding to or involving a spectral synthesis, wherein the result of the spectral synthesis leads to the baseline and ripple signals.

The reliability determining unit can be configured to carry out a similar spectral analysis and/or synthesis based on the ventilation rate VR and the pulse rate PR to identify the reliability determination signals ripple_up2 and ripple_down2 in the signals env_up and env_down, respectively. For instance, a single spectral analysis and/or synthesis can be carried out by a unit combining the functions described above with respect to the fluid responsiveness parameter determining unit 105 and the reliability determining unit 505 in order to obtain all baseline and ripple signals, i.e. baseline_up, baseline_down, ripple_up, ripple_down, ripple_up2 and ripple_down2.

The fluid responsiveness parameter determining unit 105 and/or the reliability determining unit 505, which may be the same unit or different units as indicated above, are preferably configured to compute further signals baseline_sum, ripple_sum and ripple_sum2 in accordance with the following equations:

$$\text{baseline\_sum} = \text{baseline\_up} + \text{baseline\_down} , \qquad (11)$$

$$\text{ripple\_sum} = \text{ripple\_up} + \text{ripple\_down} , \qquad (12)$$

$$\text{ripple\_sum2} = \text{ripple\_up2} + \text{ripple\_down2} \qquad (13)$$

Hence, the "sum" signals on the left-hand side of the above equations are computed by an addition of the respective baseline and ripple signals. These "sum" signals baseline_sum, ripple_sum and ripple_sum2 and/or a subset thereof are used for determining the fluid responsiveness parameter FRP and its reliability Q.

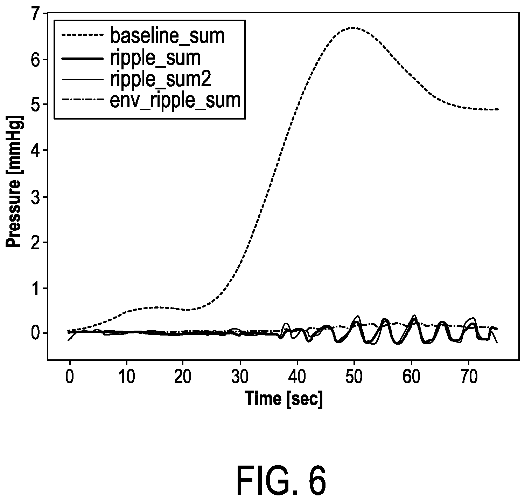

Particularly the fluid responsiveness parameter determining unit 105 can further be configured to compute the signal env_ripple_sum from ripple_sum by applying a filter. As already indicated above, this filter can correspond to the asymmetric filter described above for obtaining the envelope signals env_up and env_down, or can be a different filter. For instance, the signal env_ripple_sum can be obtained by providing the signal ripple_sum or the absolute value of the signal ripple_sum as input to the filter described with respect to equation (8), wherein now as time constants for the filter $T_{up} = T_{resp}/4$ and $T_{down} = T_{resp} \cdot 4$ are used, with $T_{resp} = 60 / VR$ and VR referring to the ventilation rate measured in respirations per minute. The output of this procedure of taking the signal ripple_sum or its absolute value and then applying the adapted filter is then assumed as the signal env_ripple_sum. Fig. 6 shows schematically and exemplarily the "sum" signals as well as the signal env _ripple_sum. The same procedure as just described for computing env_ripplesum from ripple_sum may also be applied by the fluid responsiveness parameter determining unit 105 to compute the signal env _ripple_up from ripple_up. In particular, the same filter, with the same parameters, may be used, wherein this filter is then applied to ripple_up or its absolute value to obtain env_ripple_up. In a variant, the filters used for computing env_ripple_sum from ripple_sum and for computing env_ripple_up from ripple_up may differ in the choice of parameters or altogether.

As will be subsequently described by reference to two specific exemplary fluid responsiveness parameters, the fluid responsiveness parameter determining unit 105 is configured to determine a fluid responsiveness parameter based on characteristic values of the first fluid responsiveness parameter determination signals baseline_up and baseline_down, particularly their sum baseline_sum, as well as one of the filtered fluid responsiveness parameter determination signals env_ripple_up and env_ripple_sum.

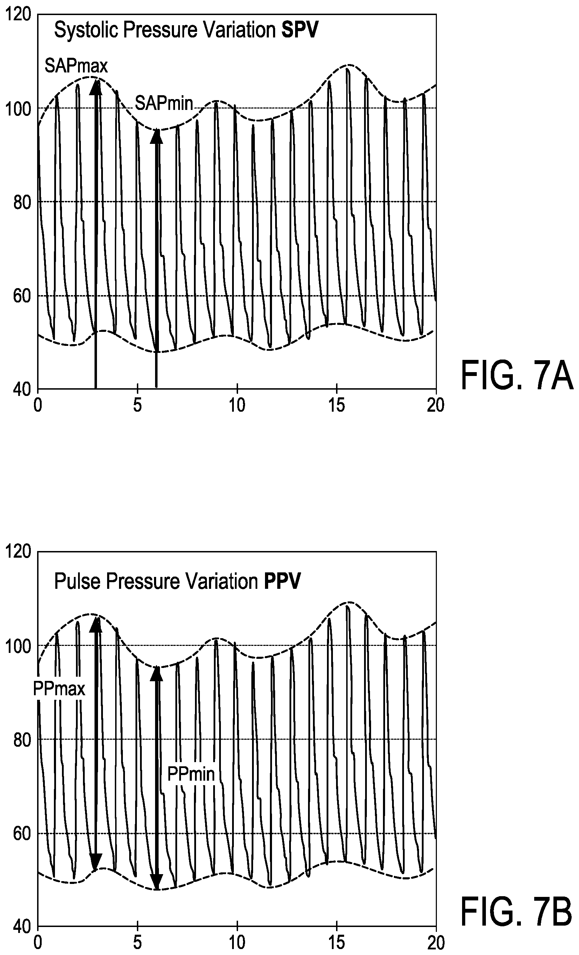

According to one example, the system 100 can be used for determining a systolic pressure variation SPV as a fluid responsiveness parameter. The parameter SPV is schematically and exemplarily illustrated in Fig. 7A, which shows a series of measured blood pressure pulses, i.e. a signal like TPac, wherein the horizontal axis measures time in units of seconds and the vertical axis measures pressure in units of mmHg. SPV can be conventionally defined as

$$SPV = \frac{SAPmax - SAPmin}{(SAPmax + SAPmin)/2} \, , \quad (14)$$

where SAPmin is the minimum systolic arterial pressure and SAPmax is the maximum systolic arterial pressure, as indicated in Fig. 7A. A different definition of SPV that is often used does not use the normalization by the average SAP value (SAPmax + SAPmin)/2. As these definitions suggest, conventional methods for estimating a fluid responsiveness via SPV use pressure pulses (either measured invasively or non-invasively), and particularly several pressure pulses separately, to assess variations of the pulse heights of the pressure pulses due to the respiration cycles, which are controlled via the ventilator, for instance.

In contrast, for the purpose of determining systolic pressure variation, the processed signal determining unit 104 is configured to determine, as a first processed signal, the signal env_up as described above, namely by applying the respective filter of equation (8) to the oscillatory component TPac of the blood pulsation signal TP, and to determine, as a second processed signal, the signal env_down as described above, namely by inverting the oscillatory component TPac and applying the filter of equation (8) to the inverted oscillatory component, wherein the inversion refers to a change of sign. Furthermore, the fluid responsiveness parameter determining unit 105 is configured to determine the fluid responsiveness parameter, i.e. in this case SPV, by identifying a first fluid responsiveness parameter determination signal baseline_up for the first processed signal env_up and a further first fluid responsiveness parameter determination signal baseline_down for the second processed signal env_down, combining the first fluid responsiveness parameter determination signals baseline_up and baseline_down to the combined first fluid responsiveness parameter determination signal baseline_sum by addition as shown in equation (11), identifying the second fluid responsiveness parameter determination signal ripple_up for the first processed signal env_up, and determining the filtered fluid responsiveness parameter determination signal env_ripple_up by applying the respective filter of equation (8) with the parameters described above in this regard to the second fluid responsiveness parameter determination signal ripple_up identified for the first processed signal env_up. Since, for the purpose of determining SPV, the filter is applied specifically to the second fluid responsiveness parameter determination signal ripple_up, the resulting specific filtered fluid responsiveness parameter determination signal env_ripple up can also be referred to as a filtered second fluid responsiveness parameter determination signal. The fluid responsiveness parameter determining unit 105 is configured to determine the SPV value based on the combined first fluid responsiveness parameter determination signal baseline_sum and the filtered fluid responsiveness parameter determination signal env_ripple_up, i.e. the filtered second fluid responsiveness parameter determination signal.

For the purpose of SPV determination, the system 100 further comprises in this case a blood pressure characteristic providing unit for providing the blood pressure characteristics SAP and DAP, i.e. a systolic arterial pressure (SAP) and a diastolic arterial pressure (DAP), wherein the fluid responsiveness parameter determining unit is configured to determine the SPV parameter based further on these blood pressure characteristics SAP and DAP.

The SPV parameter is computed by the fluid responsiveness parameter determining unit 105 via a computation of a modulation index, wherein the modulation index is computed by detecting the time index $k$ corresponding to the maximum peak in the signal baseline_sum and using the corresponding time index in the signal env _ripple_up, i.e. in the "envelope" of ripple_up. In particular, the SPV parameter is determined in accordance with the following equation:

$$SPV = 2 \cdot \frac{\displaystyle\max_{k-\frac{N}{2} \le n \le k+\frac{N}{2}} (\text{env\_ripple\_up}[n])}{\text{baseline\_sum[k]}} \cdot \frac{SAP - DAP}{SAP} , \quad (15)$$

where $N$ is a time window (in number of samples, or sampling points) that can be, e.g., a few respiration cycles long, such that a plurality of samples are used in determining the maximum of the env_ripple_up signal, and with SAP being the average systolic pressure and DAP being the average diastolic pressure. The factor of 2 enters the above equation because the signal env_ripple_up only represents an upper envelope of ripple_up. The factor (SAP-DAP) / SAP is required because the use of the "baseline" and "ripple" only reflects the pulse-pressure (PP) variation (PP = SAP-DAP), whereas the definition of SPV would require a normalization with SAP. The SAP and DAP values can be obtained from a non-invasive blood pressure (NIBP) measurement as described, for instance, in WO 2020/148137 A1, which is herewith incorporated by reference in its entirety.

The system 100 can also be used for determining a pulse pressure variation PPV as a fluid responsiveness parameter, which constitutes a second example. To give an example of the clinical meaning of PPV as a fluid responsiveness parameter, a PPV of 12 % or more is typically taken as an indication that a patient would need more fluid. The PPV is schematically and exemplarily illustrated in Fig. 7B, which again shows a series of measured blood pressure pulses, i.e. a signal like TPac, wherein the horizontal axis measures time in units of seconds, and the vertical axis measures pressure in units of mmHg. PPV is conventionally defined as

$$PPV = \frac{\text{PPmax} - \text{PPmin}}{(\text{PPmax} + \text{PPmin})/2} , \quad (16)$$

where PPmin is the minimum pulse height (pulse pressure) and PPmax is the maximum pulse height, as indicated in Fig. 7B.

Hence, while SPV estimates FRP with a focus on the maxima of the pressure pulses, i.e. the systolic arterial pressure, PPV estimates the fluid responsiveness in terms of the ratio between pulse height variation due to respiration, or ventilation, and the average pulse height. But still, as usual, pressure pulses (either measured invasively or non-invasively), and particularly several pressure pulses separately, are used to assess variations of the pulse heights of the pressure pulses due to the respiration cycles.

In contrast, for the purpose of determining PPV, the processed signal determining unit 104 is configured to determine, as a first processed signal, the signal env_up by applying, as described above, the respective filter of equation (8) to the oscillatory component TPac of the blood pulsation signal TP, and to determine, as a second processed signal, the signal env_down as also described above, namely by first inverting the oscillatory component TPac and applying the filter to the inverted oscillatory component. The fluid responsiveness parameter determination unit 105 is configured to determine the PPV parameter by identifying the first fluid responsiveness parameter determination signal for the first processed signal env_up and a further first fluid responsiveness parameter determination signal for the second processed signal env_down, and to combine the two first fluid responsiveness parameter determination signals to the combined first fluid responsiveness parameter determination signal baseline_sum by addition as shown in equation (11), wherein the fluid responsiveness parameter determining unit 105 is further configured to identify the second fluid responsiveness parameter determination signal ripple_up for the first processed signal env_up, to identify the further second fluid responsiveness parameter determination signal ripple_down for the second processed signal env_down, and to combine the two second fluid responsiveness parameter determination signals ripple_up and ripple _down to a combined second fluid responsiveness parameter determination signal ripple_sum by addition as shown in equation (12), and furthermore to determine the filtered combined second fluid responsiveness parameter determination signal env_ripple_sum by applying the respective filter to the combined second fluid responsiveness parameter determination signal ripple_sum. The fluid responsiveness parameter determination unit 105 is configured to determine the PPV parameter based on the combined first fluid responsiveness parameter determination signal baseline_sum and the filtered combined second fluid responsiveness parameter determination signal env_ripple_sum.

Also the PPV parameter is computed by the fluid responsiveness parameter determination unit 105 via a computation of a modulation index, wherein in this case the modulation index is computed by detecting the time index $k$ corresponding to the maximum peak in the signal baseline_sum and using the corresponding time index in the signal env_ripple_sum, i.e. in the "envelope" of ripple_sum instead of ripple_up. In particular, the PPV parameter is determined in accordance with the following equation:

$$PPV = 2 \cdot \frac{\max\limits_{k-\frac{N}{2} \le n \le k+\frac{N}{2}} (\text{env\_ripple\_sum}[n])}{\text{baseline\_sum}[k]} \, , \quad (17)$$

where *N* is again a time window (in number of samples, or sampling points) that can be, e.g., a few respiration cycles long, such that a plurality of samples are used in determining the maximum of the env_ripple_sum signal.

Hence, the computations carried out to determine the SPV parameter and the PPV parameter only differ in the extra factor (SAP-DAP) / SAP for SPV and in the choice between env _ripple_up and env_ripple_sum for determining the respective modulation index. In a variant, this extra factor could also be omitted for determining SPV.

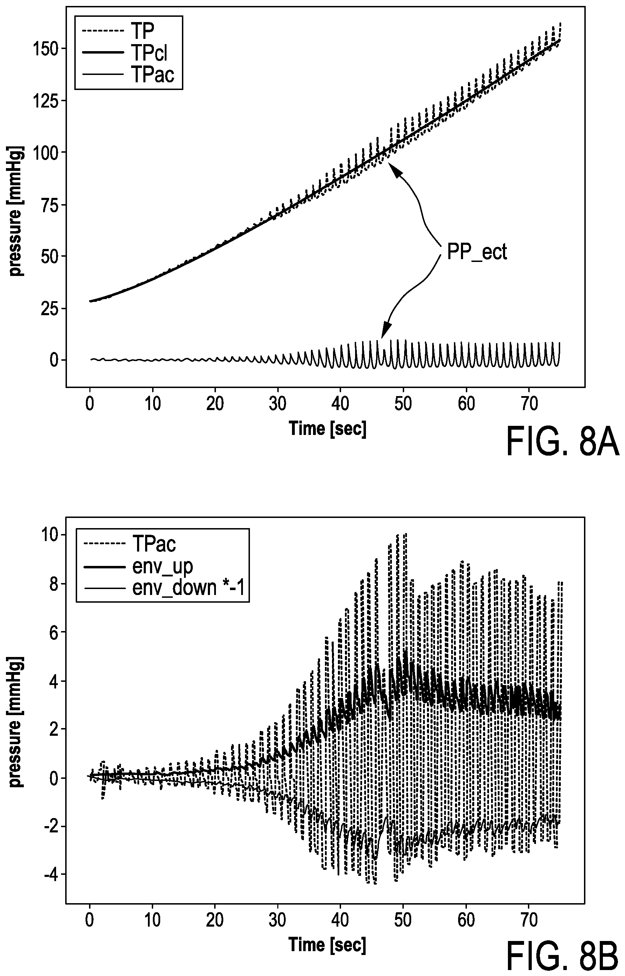

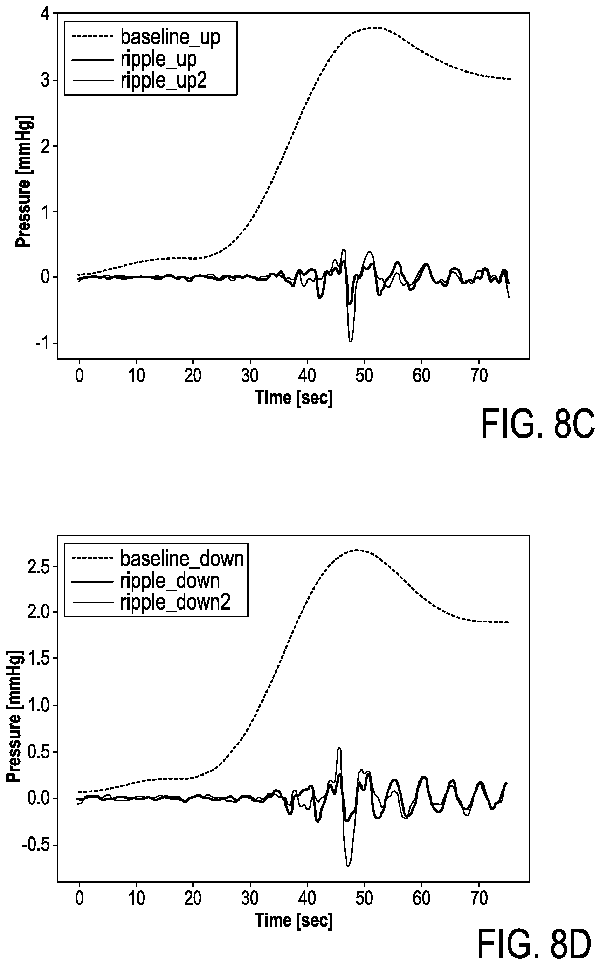

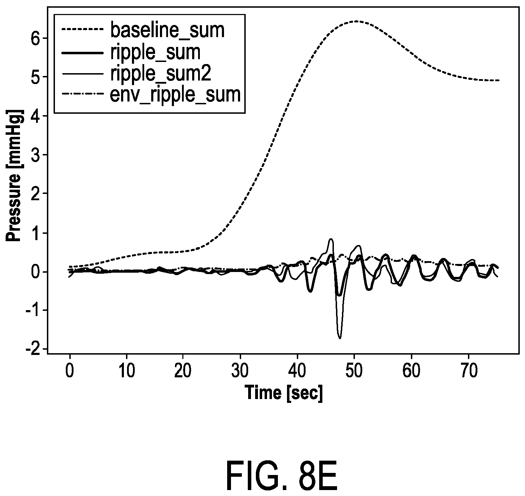

As schematically and exemplarily illustrated in Fig. 8A, the blood pulsation signal TP can comprise blood pulses, or pressure pulses indicative of blood pulses, that are due to ectopic beats. Such pulses may be considered as artefacts in the signal. In Fig. 8A, a pulse due to an ectopic beat is indicated in the blood pulsation signal TP and its oscillatory component TPac by PP_ect. The indicated pulse is attenuated by 50 %. Pulses due to ectopic beats can carry over to any signals derived from the blood pulsation signal TP, as schematically and exemplarily illustrated in Figs. 8B, 8C, 8D and 8E, which show signals corresponding to the signals shown in Figs. 2, 3, 4A and 4B, respectively, but determined based on the blood pulsation signal TP comprising pulses due to an ectopic beat as shown Fig. 8A instead of the blood pulsation signal TP without any pulse due to an ectopic beat shown in Fig. 2. Hence, the accuracy of the fluid responsiveness parameter determination can suffer from the presence of pulses corresponding to ectopic beats in the blood pulsation signal TP. In order to diminish the detrimental effect of ectopic beats on the accuracy of FRP determination, the processed signal determining unit 104 can determine the processed signals env_up and env_down based on the blood pulsation signal TP by also applying a regularization of detected blood pulses in the blood pulsation signal TP that are due to ectopic beats. For instance, the processed signal determining unit 104 can comprise a dedicated ectopic beats detection unit configured to detect the blood pulses in the blood pulsation signal TP that are due to ectopic beats.

The regularization of detected blood pulses due to ectopic beats, which could also be regarded as a reparation or fixing of the blood pulsation signal TP, can ensure that no alleged respiration modulation is considered in determining the fluid responsiveness parameter that is actually only caused by ectopic beats. The pulses due to ectopic beats can be detected based on temporal and amplitude information, wherein then the respective missing or weak pulse may be artificially filled in based on a previous or subsequent pulse in the signal. Some ectopic beat induced pulses, particularly those that are not fully attenuated like the one indicated in Fig. 8A, may be repaired only with more efforts than others. Preferably, only those pulses are detected as being due to ectopic beats and thereafter regularized that are weakened beyond a predetermined degree, i.e. only severely weak pulses, since it should be avoided that pulses are modified that are only weakened due to respiration modulation. Also any of the methods described in the article "Predicting fluid responsiveness with stroke volume variation despite multiple extrasystoles" by M. Cannesson et al., Critical Care Medicine, Volume 40, Issue 1, pages 193 to 198 (2012) may be used to improve the situation of ectopic beats, particularly for the regularization of detected blood pulses in the blood pulsation signal due to ectopic beats, wherein this article is incorporated herewith by reference in its entirety.

The regularization of pulses in the signal TP due to ectopic beats is optional, but it can ensure a stable pulse rate in the signal, i.e. which may be particularly advantageous for longer measurement times, i.e. if a blood pulsation signal is used for FRP determination that has been acquired over a relatively long time window. For patients having heartbeats that show real arrhythmia, a regularization can be challenging or even impossible, in which case it may be preferred to not trust the determined fluid responsiveness parameter, i.e. associate zero reliability to it. For instance, as described in WO 2019/211210 A1, which is herewith incorporated by reference in its entirety, a heart rate variability metric (such as the one called PRvar) can be used to decide whether there are arrhythmias. If this is the case, the estimated fluid responsiveness parameters might not be used to improve the SV estimation (and instead an assumed fluid responsiveness value may be used).

More generally, the reliability determining unit 505, which can, as already described above, be identical to or integrated in the fluid responsiveness parameter determining unit 105, can be configured to determine the reliability of the determined SPV parameter and the determined PPV parameter in terms of a respective quality index QI. In particular, the quality index for the determined SPV parameter, referred to herein as SPV QI, can be computed in accordance with the following equation:

$$\text{SPV QI} = 1 - \frac{\sum_{n=k-N/2}^{k+N/2} |\text{ripple\_up}[n] - \text{ripple\_up2}[n]|}{\sum_{n=k-\frac{N}{2}}^{k+\frac{N}{2}} |\text{ripple\_up}[n] + \text{ripple\_up2}[n]|} \, , \quad (18)$$

wherein $k$ and $N$ are defined as in the above equation (8) for determining the SPV parameter.

Moreover, the quality index for the determined PPV parameter, referred to herein as PPV QI, can be computed in accordance with the following equation:

$$PPV\ QI = 1 - \frac{\sum_{n=k-\frac{N}{2}}^{k+\frac{N}{2}} |\ ripple\_sum[n]\ - ripple\_sum2[n]\ |}{\sum_{n=k-\frac{N}{2}}^{k+\frac{N}{2}} |\ ripple\_sum[n]\ + ripple\_sum2[n]\ |} \ . \quad (19)$$

wherein $k$ and $N$ are defined as in the above equation (10) for determining the PPV parameter.

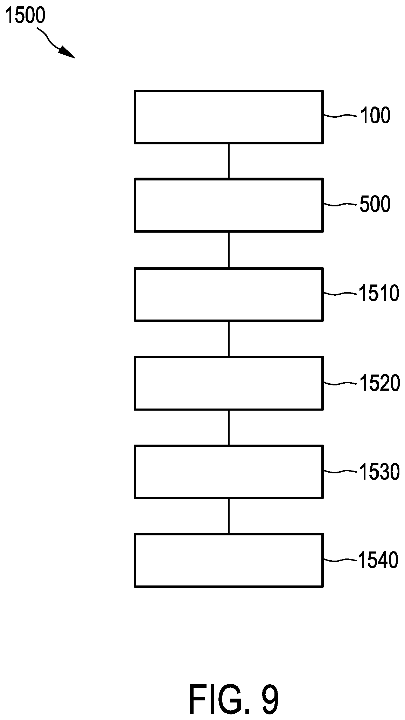

Fig. 9 shows schematically and exemplarily a system 1500 for determining, as a hemodynamic parameter correlated with a fluid responsiveness parameter, a stroke volume SV for a patient. The system 1500 comprises the system 100 for determining the fluid responsiveness parameters SPV and PPV for the patient, and the system 500 for determining, in terms of the quality indices SPV QI and PPV QI, the respective reliabilities of the fluid responsiveness parameters SPV and PPV determined by the system 100. The system 1500 further comprises a first model providing unit 1510 that is configured to provide a first model of the stroke volume SV depending on SPV and PPV, and a second model providing unit 1520 configured to provide a second model of the stroke volume SV that does not depend on SPV and PPV. Furthermore, the system 1500 comprises a combined model determining unit 1530 that is configured to determine a combined model based on the first model, the second model, and the respective one of the quality indices SPV QI and PPV QI, and a hemodynamic parameter determining unit 1540 configured to determine the stroke volume SV by applying the combined model.

In particular, a pulse contour stroke volume (PCSV) can be used as a measure of the stroke volume SV. Thus, the first model can correspond to a first function SV1 expressing the pulse contour stroke volume PCSV in terms of any of SPV and PPV, and the second model can correspond to a second function SV expressing the pulse contour stroke volume PCSV in terms of other quantities than SPV and PPV.

An example of the first model to compute stroke volume with fluid responsiveness parameters is mentioned in Eq. (31) of WO 2019/211210 A1, whereas an example of the second model to compute stroke volume without fluid responsiveness parameters is mentioned in Eq. (11) of WO 2019/211210 A1. Alternatively, further examples of the second model are known from the Wesseling Algorithm or alternative methods, see Table 1 in the contribution "The Effect of Signal Quality on Six Cardiac Output Estimators" by T. Chen et al., 36th Annual Computers in Cardiology Conference, pages 197 to 200 (2009), which is herewith incorporated by reference in its entirety.

The combined model can then correspond to a function expressing the pulse contour stroke volume PCSV in terms of the first function SV1, the second function SV2, and a mapping function w in accordance with the following equation:

$$PCSV = w \cdot SV1 + (1 - w) \cdot SV2, \quad (20)$$

wherein the mapping function can have the following form:

$$w = QI, \quad (21)$$

with QI being SPV QI if SV1 is chosen to depend on SPV and being PPV QI if SV1 is chosen to depend on PPV. Also a combination of PPV and SPV is possible by applying a weighted combination of the PPV and the SPV parameter for SV1 together with a weighted combination of PPV QI and SPV QI for w like done in Eq. (16) of WO 2019/211210 A1. Alternatively, other, non-linear mapping functions w can be used, such as sigmoid functions, for instance.

Hence, the respective FRP is used for the estimation of a first stroke volume SV1 expressed by the first function, but there is a fall-back scenario where the FRP is not used to estimate the stroke volume, which is then a second stroke volume SV2 expressed by a second function. The quality index determined by the system 500 for the respectively used FRP enters the stroke volume determination via the mapping-function w, which could also be regarded as giving a weighting parameter that weights SV1 and SV2 in order to compute PCSV as the final stroke volume output.

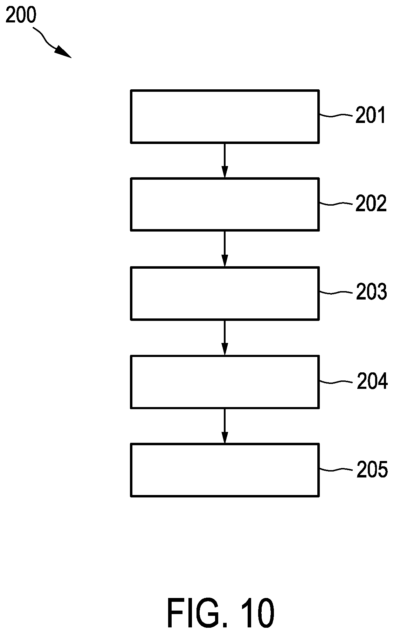

Fig. 10 shows schematically and exemplarily a method 200 for determining a fluid responsiveness parameter for a patient. The method 200 comprises a step 201 of providing a respiratory rate for the patient, a step 202 of providing a heart rate for the patient and a step 203 of providing the blood pulsation signal TP for the patient, wherein the blood pulsation signal TP is a measured signal indicative of a series of blood pulses of the patient as schematically and exemplarily illustrated in Fig. 2, for instance. The method 200 further comprises a step 204 of determining one or more processed signals based on the blood pulsation signal TP, wherein, as described in detail above, the processed signals are indicative of respective envelopes of the blood pulsation signal TP. Furthermore, the method 200 comprises a step 205 of determining the fluid responsiveness parameter. In this step 205, which could also be regarded as comprising several sub-steps, one or more first fluid responsiveness parameter determination signals are determined based on the one or more processed signals, wherein the one or more first fluid responsiveness parameter determination signals each correspond to a respective one of the one or more processed signals in a spectral range up to the respiratory rate, and one or more second fluid responsiveness parameter determination signals are determined based on the one or more processed signals, wherein the one or more second fluid responsiveness parameter determination signals each correspond to a respective one of the one or more processed signals at the respiratory rate and any of its harmonics up to the heart rate. In the course of step 205, then, the fluid responsiveness parameter is determined based on the one or more first fluid responsiveness parameter determination signals and the one or more second fluid responsiveness parameter determination signals.

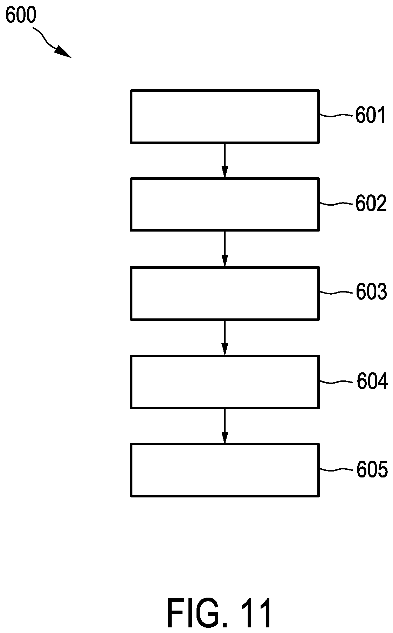

Fig. 11 shows schematically exemplarily a method 600 for determining a reliability of a fluid responsiveness parameter determined by the method shown in Fig. 10. The method 600 comprises a step 601 of providing the respiratory rate based on which the fluid responsiveness parameter has been determined, a step 602 of providing the heart rate based on which the fluid responsiveness parameter has been determined, a step 603 of providing the one or more processed signals based on which the fluid responsiveness parameter has been determined, and a step 604 of providing the one or more second fluid responsiveness parameter determination signals corresponding to the one or more processed signals. In a further step 605 of the method 600, which could also be regarded as comprising several substeps, the reliability of the determined fluid responsiveness parameter is determined by identifying one or more reliability determination signals based on the one or more processed signals, wherein the one or more reliability determination signals correspond to a respective one of the one or more processed signals in a spectral range between the respiratory rate and the heart rate, and the reliability of the determined fluid responsiveness parameter is determined based on the one or more second fluid responsiveness parameter determination signals as well as the one or more reliability determination signals.

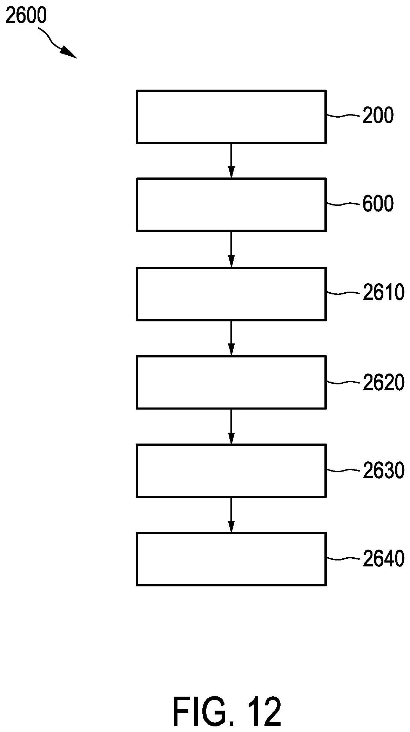

Fig. 12 shows schematically and exemplarily a method 2600 for determining a hemodynamic parameter correlated with a fluid responsiveness parameter as determined by the method 200 shown in Fig. 10. The method 2600 comprises the steps of the method 200 by which a fluid responsiveness parameter is determined for a patient, and the steps of the method 600 by which a reliability of the determined fluid responsiveness parameter is determined. Furthermore, the method 2600 comprises a step 2610 of providing a first model of the hemodynamic parameter to be determined, wherein the first model depends on the fluid responsiveness parameter determined in accordance with the method 200, and a step 2620 of providing a second model of the hemodynamic parameter, wherein the second model differs from the first model in that it does not depend on the fluid responsiveness parameter. Moreover, the method 2600 comprises a step 2630 of determining a combined model based on the first model, the second model and the determined reliability of the fluid responsiveness parameter, i.e. the reliability determined in accordance with the method 600. In a further step 2640 of the method 2600, the hemodynamic parameter is determined by applying the combined model.

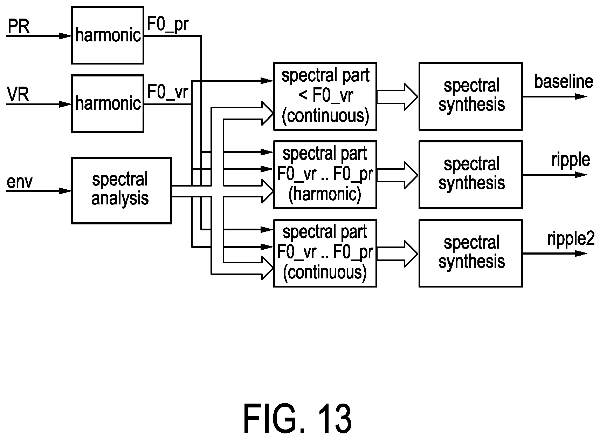

Fig. 13 shows schematically and exemplarily how the "baseline", "ripple" and "ripple2" signals baseline_up, baseline_down, ripple_up, ripple_down and ripple_up2 as well as ripple_down2, which are examples of the first and second fluid responsiveness parameter determination signals and the reliability determination signals, respectively, are determined based on a pulse rate PR, understood as being indicative of the heart rate, a provided ventilation rate VR, which controls the respiration rate of the patient, and the processed, "envelope" signals env_up and env_down ("env") determined based on the measured blood pulsation signal. It is shown that the harmonics F0_pr and F0_vr of the pulse rate PR and the ventilation rate VR are determined, and a corresponding spectral analysis is carried out on the processed signals in order to determine their spectral parts a) below the ventilation rate, b) at the ventilation rate and its harmonics up to the pulse rate and c) between the ventilation rate and the pulse rate. Based on the several spectral parts, the baseline and ripple signals are determined by a spectral synthesis, as indicated by the rightmost blocks in Fig. 13.

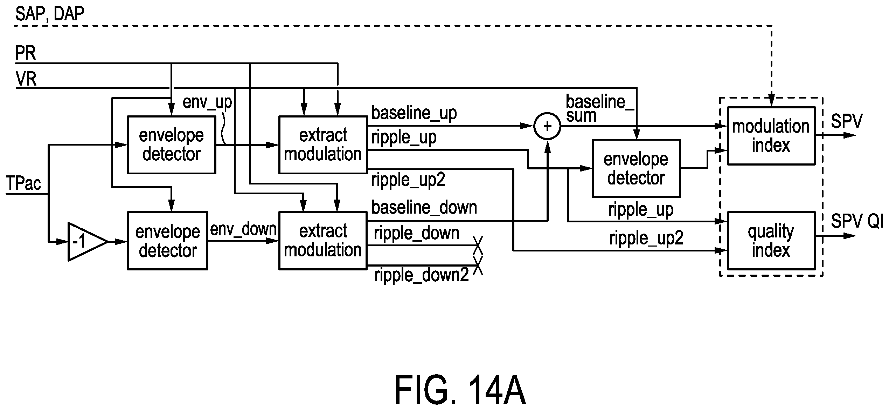

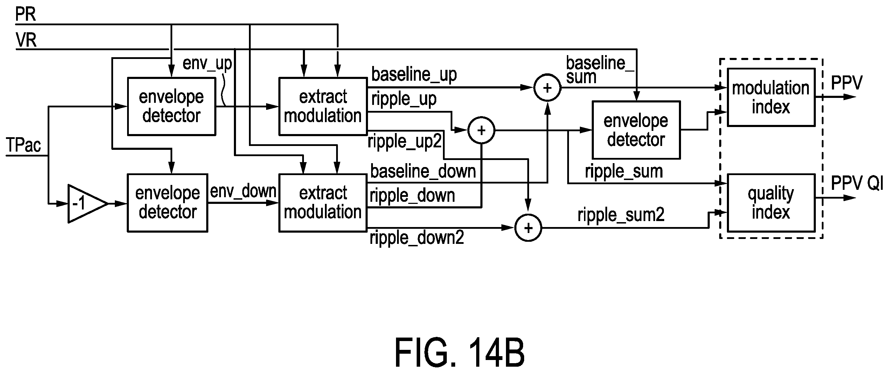

Figs. 14A and 14B shows schematically and exemplarily how the fluid responsiveness parameters SPV and PPV and their respective reliabilities can be determined. As shown in Fig. 14A, in order to determine SPV and its reliability SPV QI, the signals env_up and env_down are determined based on the oscillatory component TPac of the blood pulsation signal TP via processing in what might also be referred to as an envelope detector, wherein based on the signals env_up and env_down, the signals baseline_up, ripple_up, ripple_up2 and baseline_down are determined, but not the signals ripple_down and ripple_down2, since these latter two signals are not needed for determining SPV and its reliability SPV QI. The determination of the selected baseline and ripple signals can also be regarded as an extraction of a modulation of the pulses in the blood pulsation signal, as indicated by the corresponding blocks in Fig. 14A. As also illustrated, the pulse rate PR and the ventilation rate VR enter the envelope detection and modulation extraction, such as via the parameters of the filter used for envelope detection and the choice of the spectral parts in the spectral analysis performed for modulation extraction. Notably, VR does not enter the detection of env_up and env_down in this case.

The SPV parameter is then determined based on the signal baseline_sum formed from the signals baseline_up and baseline_down by addition, and by an envelope, again determined by the envelope detector by use of a filter, for instance, of the ripple_up signal, via a determination of a corresponding modulation index optionally depending also on systolic arterial pressure SAP and diastolic arterial pressure DAP. The reliability of the SPV parameter is determined via a corresponding quality index depending on the signals ripple_up and ripple_up2. While VR does not enter the detection of env_up and env_down in this case, PR does not enter the detection of the envelope for ripple_up.

As seen in Fig. 14B, the determination of PPV and its reliability differs slightly from the determination of SPV and its reliability. In particular, also ripple_down and ripple_down2 are determined, and corresponding "sum" signals ripple_sum and ripple_sum2 are determined by addition of the respective ripple signals. PPV is then determined based on an envelope detected for ripple_sum and further based on the signal baseline_sum, but without reference to SAP and DAP. The reliability of PPV is determined based on the two "summed" ripple signals instead of only the signals ripple_up and ripple_up2. While VR does not enter the detection of env_up and env_down, PR does not enter the detection of the envelope for ripple_sum in this case.

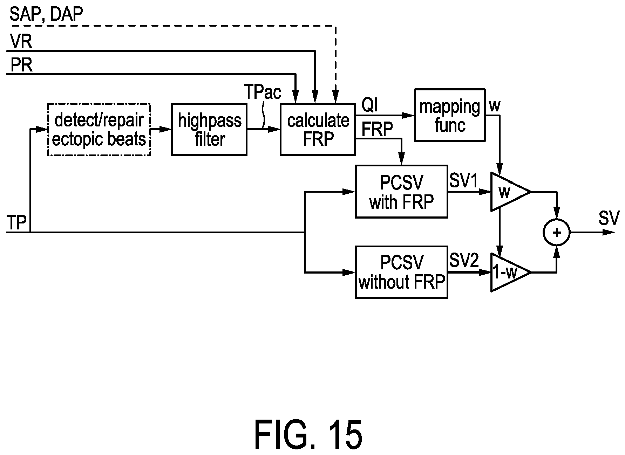

Fig. 15 shows schematically and exemplarily how a stroke volume SV is determined as an example of a hemodynamic parameter based on any of the fluid responsiveness parameters SPV and PPV. Since systolic arterial pressure and diastolic arterial pressure are only optionally needed as input data in case SPV is used as fluid responsiveness parameter FRP, the corresponding arrow in Fig. 15 is dashed, indicating optionality. Similarly, as has been described in further detail above, the detection of those pulses in the blood pulsation signal, i.e. the tissue pressure signal TP, for instance, that are due to ectopic beats, and their regularization, or reparation, is optional even in the already quite specific embodiment illustrated, as indicated by the frame of the corresponding box. According to Fig. 15, the oscillatory component of TP is determined by applying a highpass filter. The respective fluid responsiveness parameter and its reliability is then determined as described above with respect to Figs. 13 as well as 14A and 14B. The mapping function was well as the first model function SV1 and the second model function SV2 described further above are used to compute the stroke volume SV.

The fluid responsiveness parameter (FRP) is an important clinical parameter in the surgery setting and sometimes also used as intermediate parameter in stroke volume estimation algorithms (like those used with a system as described in EP 3 430 992 A1). The FRP estimation aims to measure the modulation-index of respiration influence on heart pulsations. However, in situations where it is desired to quickly provide the FRP to the clinician (like also in spot-check scenarios), there will be only a limited amount of respiration cycles that can be exploited. Consequently, the performance of FRP determination is extremely sensitive to artefacts and ectopic beats. Herewith, robust means are provided to compute the FRP with a quality index by using apriori inputs of a respiratory rate, like a ventilation rate (VR), and a heart rate, such as indicated by a pulse rate (PR). Both VR and PR can be safely assumed to be constant, since for VR we always have a fixed rate in surgery settings and for PR we can detect and fix ectopic beats.

In other words, the systems and methods described herein can be used for a short duration measurement of fluid responsiveness parameters together with a respective quality index, which is considered very important in surgery settings. It has been found that for short duration measurements, the fact can be exploited that there is a fixed/stable respiration rate and a fixed/stable heart rate within the corresponding short time window, at least to a sufficient degree. Such a short duration measurement can be applied to spot-check scenarios (using a system as described in EP 3 430 992 A1 or a traditional air-cuff). Furthermore, it can be applied for continuous measurements with e.g. arterial blood pressure (ABP) and (photo)plethysmography (PPG), where it is required to obtain fluid responsiveness parameters that are to be tracked quickly, e.g. during maneuvers like passive-leg raise. Hence, besides the use case that the fluid responsiveness parameter (FRP) is provided to and interpreted by the clinician, it can also be used internally in algorithms like those used with systems as known from EP 3 430 992 A1. As mentioned already, these systems are particularly useful in the surgery setting with ventilated patients. The estimated fluid responsiveness parameters (like PPV) can be used for improvement of the stroke volume (SV) estimation, but so far mainly in case of a stable heart rate (i.e., no arrhythmias).

In order to determine a good quality index that corresponds to the quality, or reliability, of the determined fluid responsiveness parameter an a priori known value of the respiratory rate, such as the VR (ventilation rate), and the heart rate, such as indicated by PR (pulse rate), can be assumed, and these rates can be exploited in the spectral domain for estimating the so-called "respiration ripple", which is the variation of the pulse pressure heights that are modulated by the respiration of the patient (induced by the ventilator, for instance). The respiratory rate can be used to extract the first and next harmonics from the spectrum that are contributing to this ripple. The heart rate can be used to decide on how many harmonics can be included in this ripple extraction, because otherwise the heart pulsations will start to influence the estimation of the ripple. The quality of the fluid responsiveness parameter can be assessed by comparing the estimated ripple (based on, for instance, the individual ventilator harmonics between VR and PR) by a ripple that is estimated by using the whole spectrum between VR and PR.

The systems and methods described herein can be used together with a measurement system as described EP 3 430 992 A1, and also the above described illustrated embodiments are based on measurement data acquired using such a system, even though they could also have been acquired with a different system. Since, when using a system as described in EP 3 430 992 A1, typically the reliability of determined fluid responsiveness parameters is of interest, it is not only envisaged to present the FRP to the clinician, but additionally a quality-index for the FRP could be provided, in order to decide on how to use the FRP in the stroke volume (SV) estimation.

The fluid responsiveness parameter (FRP) and its quality index can be preferably shown on a patient monitor, because they will be beneficial for an assessment by the clinician. Furthermore, also the ventilation rate (VR) and pulse rate (PR) can be shown on the patient monitor. In this case, the patient monitor may require corresponding input data streams.

Although the embodiments described in detail above relate to SPV and PPV as exemplary fluid responsiveness parameters, and a blood pulsation signal acquired used in a particular way, in other embodiments, other fluid responsiveness parameters, corresponding reliabilities and correlated hemodynamic parameters can be determined, particularly also based on blood pulsation signals acquired using different measurement systems, such as via the pressure of an air-filled cuff used for non-invasive blood pressure measurement or by (photo-)plethysmography measurement (PPG) or via a displacement measurement using an inertial measurement unit (IMU). Moreover, even for determining SPV and PPV, their reliabilities and SV based thereon, other equations than those given above may be found. For instance, different types of filters than the one defined in equation (1) may be used.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the providing of a respiratory rate, a heart rate, a blood pulsation signal or any other signal, as well as any identifying and determining of signals based on other signals, et cetera, performed by one or several units, can be performed by any other number of units. These procedures can be implemented as program code of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

A system for FRP determination is presented that comprises units for providing a respiratory rate, a heart rate and a measured blood pulsation signal indicative of a series of blood pulses of a patient. The system further comprises a unit for determining, based on the blood pulsation signal, a processed signal indicative of an envelope of the blood pulsation signal, and a unit for determining the FRP by a) identifying, based on the processed signal, a first FRP determination signal corresponding to the processed signal in a spectral range up to the respiratory rate, and a second FRP determination signal corresponding to the processed signal at the respiratory rate and any of its harmonics up to the heart rate, and b) determining the FRP based on the first and the second FRP determination signal.

## Claims

1. A system (100) for determining a fluid responsiveness parameter for a patient, wherein the determined fluid responsiveness parameter is a systolic pressure variation (SPV) or a pulse pressure variation (PPV), wherein the system comprises:

   - a respiratory rate (101) providing unit configured to provide a respiratory rate,
   - a heart rate providing unit (102) configured to provide a heart rate,
   - a blood pulsation signal providing unit (103) configured to provide a blood pulsation signal, wherein the blood pulsation signal is a measured signal indicative of a series of blood pulses of the patient,
   - a processed signal determining unit (104) configured to determine a first processed signal (env_up) based on the blood pulsation signal, wherein the first processed signal is indicative of an upper envelope of the blood pulsation signal, and a second processed signal (env_down) based on the blood pulsation signal, wherein the second processed signal is indicative of a lower envelope of the blood pulsation signal, and
   - **characterized in that** the system further comprises a fluid responsiveness parameter determining unit (105) configured to determine the fluid responsiveness parameter (SPV, PPV) by:

     a) identifying first fluid responsiveness parameter determination signals (baseline_up, baseline__down) based on the first and second processed signals (env_up, env_down), wherein the first fluid responsiveness parameter determination signals correspond to the first and second processed signals (env_up, env _down) in a spectral range up to the respiratory rate, and identifying second fluid responsiveness parameter determination signals (ripple_up, ripple _down) based on the first and second processed signals (env_up, env_down), wherein the second fluid responsiveness parameter determination signals (ripple_up, ripple_down) correspond to the first and second processed signals (env_up, env_down) at the respiratory rate and any of its harmonics up to the heart rate, and

b) determining the fluid responsiveness parameter (SPV, PPV) based on the first fluid responsiveness parameter determination signals (baseline_up, baseline_down) and the second fluid responsiveness parameter determination signals (ripple_up, ripple_down).

2. The system (100) as defined in claim 1, wherein the processed signal determining unit (104) is configured to obtain the first and second processed signals (env_up, env_down) by applying a filter to an oscillatory component of the blood pulsation signal, wherein the fluid responsiveness parameter determining unit (105) is configured to determine a filtered fluid responsiveness parameter determination signal (env_ripple_up, env_ripple_sum) based on the second fluid responsiveness parameter determination signals (ripple_up, ripple_down) and a further application of a filter, and to determine the fluid responsiveness parameter (SPV, PPV) based on the first fluid responsiveness parameter determination signals (baseline_up, baseline_down) and the filtered fluid responsiveness parameter determination signal (env_ripple_up, env_ripple_sum).

3. The system (100) as defined in claim 2, wherein the fluid responsiveness parameter determining unit (105) is configured to determine the fluid responsiveness parameter (SPV, PPV) based on characteristic values of the first fluid responsiveness parameter determination signals (baseline_up, baseline_down) and the filtered fluid responsiveness parameter determination signal (env_ripple_up, env_ripple_sum).

4. The system (100) as defined in any of the preceding claims, wherein the processed signal determining unit (104) is configured to determine a first processed signal (env_up) obtained by applying a filter to an oscillatory component of the blood pulsation signal and a second processed signal (env_down) obtained by inverting the oscillatory component and applying the filter to the inverted oscillatory component, and wherein the fluid responsiveness parameter determining unit (105) is configured to determine the fluid responsiveness parameter (SPV) by:

a') identifying a first fluid responsiveness parameter determination signal for each of the first processed signal (env_up) and the second processed signal (env_down) and combining the first fluid responsiveness parameter determination signals (baseline_up, baseline_down) to a combined first fluid responsiveness parameter determination signal (baseline_sum), identifying a second fluid responsiveness parameter determination signal (ripple_up) for the first processed signal (env_up), determining a filtered fluid responsiveness parameter determination signal (env_ripple_up) by applying a filter to the second fluid responsiveness parameter determination signal (ripple_up) identified for the first processed signal (env_up), and

b') determining the fluid responsiveness parameter (SPV) based on the combined first fluid responsiveness parameter determination signal (baseline_sum) and the filtered fluid responsiveness parameter determination signal (env_ripple_up).

5. The system (100) as defined in any of the preceding claims, wherein the system further comprises:

- a blood pressure characteristic providing unit for providing a blood pressure characteristic (SAP, DAP),

wherein the fluid responsiveness parameter determining unit (105) is configured to determine the fluid responsiveness parameter (SPV) based further on the blood pressure characteristic (SAP, DAP).

6. The system (100) as defined in any of the preceding claims, wherein the processed signal determining unit (104) is configured to determine a first processed signal (env_up) obtained by applying a filter to an oscillatory component of the blood pulsation signal and a second processed signal (env_down) obtained by inverting the oscillatory component and applying the filter to the inverted oscillatory component, and wherein the fluid responsiveness parameter determining unit (105) is configured to determine the fluid responsiveness parameter (PPV) by:

a") identifying a first fluid responsiveness parameter determination signal for each of the processed signals (env_up, env_down) and combining them to a combined first fluid responsiveness parameter determination signal (baseline_sum), identifying a second fluid responsiveness parameter determination signal for each of the processed signals (env_up, env_down) and combining them to a combined second fluid responsiveness parameter determination signal (ripple_sum), and determining a filtered combined second fluid responsiveness parameter determination signal (env_ripple_sum) by further applying a filter to the combined second fluid responsiveness parameter determination signal (ripple_sum), and

b") determining the fluid responsiveness parameter (PPV) based on the combined first fluid responsiveness parameter determination signal (baseline_sum) and the filtered combined second fluid responsiveness parameter determination signal (env_ripple_sum).

7. The system (100) as defined in any of the preceding claims, wherein the determination of the first and second processed signals (env_up, env_down) based on the blood pulsation signal by the processed signal determining unit involves a regularization of detected blood pulses (PP _ect) in the blood pulsation signal that are due to ectopic beats.

8. A system (1500) for determining a hemodynamic parameter (SV) correlated with a fluid responsiveness parameter (SPV, PPV), wherein the system comprises:

- the system (100) as defined in any of claim 1 to 7,
- a respiratory rate providing unit (501) configured to provide the respiratory rate based on which the fluid responsiveness parameter (SPV, PPV) has been determined,
- a heart rate providing unit (502) configured to provide the heart rate based on which the fluid responsiveness parameter (SPV, PPV) has been determined,
- a processed signal providing unit (503) configured to provide the first and second processed signals (env_up, env _down) based on which the fluid responsiveness parameter (SPV, PPV) has been determined,
- a second fluid responsiveness parameter determination signal providing unit (504) configured to provide the second fluid responsiveness parameter determination signals (ripple_up, ripple_down) of the first and second processed signals (env_up, env _down), and
- a reliability determining unit (505) configured to determine the reliability (QI) of the determined fluid responsiveness parameter (SPV, PPV) by:

a) identifying a first reliability determination signal (ripple_up2) based on the first processed signal (env_up), and identifying a second reliability determination signal (ripple_down2) based on the second processed signal (env_down), wherein the first and second reliability determination signals (ripple_up2, ripple_down2) correspond to the first and second processed signals (env_up, env_down) in a spectral range between the respiratory rate and the heart rate, and
b) determining the reliability (QI) of the determined fluid responsiveness parameter (SPV, PPV) based on the second fluid responsiveness parameter determination signals (ripple_up, ripple_down) and the first and second reliability determination signals (ripple_up2, ripple_down2).

- a first model providing unit (1510) configured to provide a first model of the hemodynamic parameter that depends on the fluid responsiveness parameter (SPV, PPV) determined by the system (100) as defined in any of claim 1 to 7,
- a second model providing unit (1520) configured to provide a second model of the hemodynamic parameter that does not depend on the fluid responsiveness parameter (SPV, PPV), and
- a combined model determining unit (1530) configured to determine a combined model based on the first model, the second model, and the reliability (QI) of the fluid responsiveness parameter (SPV, PPV), and
- a hemodynamic parameter determining unit (1540) configured to determine the hemodynamic parameter by applying the combined model.

9. A computer-implemented method (200) for determining a fluid responsiveness parameter for a patient, wherein the determined fluid responsiveness parameter is a systolic pressure variation (SPV) or a pulse pressure variation (PPV), wherein the method (200) comprises:

- providing (201) a respiratory rate,
- providing (202) a heart rate,
- providing (203) a blood pulsation signal, wherein the blood pulsation signal is a measured signal indicative of a series of blood pulses of the patient,
- determining (204) a first processed signal (env_up) based on the blood pulsation signal, wherein the first processed signal is indicative of an upper envelope of the blood pulsation signal, and a second processed signal (env_down) based on the blood pulsation signal, wherein the second processed signal is indicative of a lower envelope of the blood pulsation signal, and **characterized in that** the method further comprises:
- determining (205) the fluid responsiveness parameter (SPV, PPV) by:

a) identifying first fluid responsiveness parameter determination signals (baseline_up, baseline__down) based on the first and second processed signals (env_up, env_down), wherein the first fluid responsiveness parameter determination signals correspond to the first and second processed signals (env_up, env_down) in a spectral range up to the respiratory rate, and identifying second fluid responsiveness parameter determination signals (ripple_up, ripple_down) based on the first and second processed signals (env_up, env_down), wherein the second fluid responsiveness parameter determination signals (ripple_up, ripple_down) correspond to the first and second processed signals (env_up, env_down) at the respiratory rate and any of its harmonics up to the heart rate, and

b) determining the fluid responsiveness parameter (SPV, PPV) based on the first fluid responsiveness parameter determination signals (baseline_up, baseline_down) and the second fluid responsiveness parameter determination signals (ripple_up, ripple_down).

10. A computer-implemented method (2600) for determining a hemodynamic parameter (SV) correlated with a fluid responsiveness parameter (SPV, PPV), wherein the method (2600) comprises:

- determining a fluid responsiveness parameter (SPV, PPV) for a patient by the method (200) as defined in claim 9,
- determining a reliability (QI) of the determined fluid responsiveness parameter (SPV, PPV) by:

- providing (601) the respiratory rate based on which the fluid responsiveness parameter (SPV, PPV) has been determined,
- providing (602) the heart rate based on which the fluid responsiveness parameter (SPV, PPV) has been determined,
- providing (603) the first and second processed signals (env_up, env_down) based on which the fluid responsiveness parameter (SPV, PPV) has been determined,
- providing (604) the second fluid responsiveness parameter determination signals (ripple_up, ripple_down) of the first and second processed signals (env_up, env_down), and
- determining (605) the reliability (QI) of the determined fluid responsiveness parameter (SPV, PPV) by:

a) identifying a first reliability determination signal (ripple_up2) based on the first processed signal (env_up), and identifying a second reliability determination signal (ripple_down2) based on the second processed signal (env_down), wherein the first and second reliability determination signals correspond to the first and second processed signals (env_up, env_down) in a spectral range between the respiratory rate and the heart rate, and

b) determining the reliability (QI) of the determined fluid responsiveness parameter (SPV, PPV) based on the second fluid responsiveness parameter determination signals (ripple_up, ripple_down) and the first and second reliability determination signals (ripple_up2, ripple_down2),

the method (2600) for determining a hemodynamic parameter (SV) correlated with a fluid responsiveness parameter (SPV, PPV) further comprising:

- providing (2610) a first model of the hemodynamic parameter that depends on the determined fluid responsiveness parameter (SPV, PPV),
- providing (2620) a second model of the hemodynamic parameter that does not depend on the fluid responsiveness parameter (SPV, PPV),
- determining (2630) a combined model based on the first model, the second model and the determined reliability (QI) of the determined fluid responsiveness parameter (SPV, PPV), and
- determining (2640) the hemodynamic parameter by applying the combined model.

11. A computer program for determining a fluid responsiveness parameter (SPV, PPV) for a patient, wherein the program comprises instructions causing the system according to claims 1-7, to carry out the steps of the method (200) as defined in claim 9, if the program is executed by the system.

12. A computer program for determining a hemodynamic parameter (SV), correlated with a fluid responsiveness parameter (SPV, PPV) determined by the computer program according to claim 11, wherein the program comprises instructions causing the system according to claim 8, to carry out the steps of the method (2600) as defined in claim 10, if the program is executed by the system.

**Patentansprüche**

1. System (100) zum Bestimmen eines Fluid-Reaktionsfähigkeitsparameters für einen Patienten, wobei der bestimmte Fluid-Reaktionsfähigkeitsparameter eine systolische Druckvariation (SPV) oder eine Pulsdruckvariation (PPV) ist, wobei das System Folgendes umfasst:

   - eine Bereitstellungseinheit (101) der Atemfrequenz, die dazu konfiguriert ist, eine Atemfrequenz bereitzustellen,
   - eine Herzfrequenz-Bereitstellungseinheit (102), die dazu konfiguriert ist, eine Herzfrequenz bereitzustellen,
   - eine Blutpulsationssignal-Bereitstellungseinheit (103), die dazu konfiguriert ist, ein Blutpulsationssignal bereitzustellen, wobei das Blutpulsationssignal ein gemessenes Signal ist, das eine Reihe von Blutpulsen des Patienten angibt,
   - eine Bestimmungseinheit (104) verarbeiteter Signale, die dazu konfiguriert ist, ein erstes verarbeitetes Signal (env_up) basierend auf dem Blutpulsationssignal zu bestimmen, wobei das erste verarbeitete Signal eine obere Hüllkurve des Blutpulsationssignals angibt, und ein zweites verarbeitetes Signal (env_down) basierend auf dem Blutpulsationssignal, wobei das zweite verarbeitete Signal eine untere Hüllkurve des Blutpulsationssignals angibt, und
   - **dadurch gekennzeichnet, dass** das System weiter eine Fluid-Reaktionsfähigkeitsparameter-Bestimmungseinheit (105) umfasst, die dazu konfiguriert ist, den Fluid-Reaktionsfähigkeitsparameter (SPV, PPV) zu bestimmen durch:

      a) Identifizieren von ersten Fluid-Reaktionsfähigkeitsparameter-Bestimmungssignalen (baseline_up, baseline_down) basierend auf dem ersten und dem zweiten verarbeiteten Signal (env_up, env_down), wobei die ersten Fluid-Reaktionsfähigkeitsparameter-Bestimmungssignale dem ersten und dem zweiten verarbeiteten Signal (env_up, env_down) in einem Spektralbereich bis zu der Atemfrequenz entsprechen, und Identifizieren von zweiten Fluid-Reaktionsfähigkeitsparameter-Bestimmungssignalen (ripple_up, ripple_down) basierend auf dem ersten und dem zweiten verarbeiteten Signal (env_up, env_down), wobei die zweiten Fluid-Reaktionsfähigkeitsparameter-Bestimmungssignale (ripple_up, ripple_down) dem ersten und dem zweiten verarbeiteten Signal (env_up, env_down) bei der Atemfrequenz und allen ihrer Harmonischen bis zu der Herzfrequenz entsprechen, und
      b) Bestimmen des Fluid-Reaktionsfähigkeitsparameters (SPV, PPV) basierend auf den ersten Fluid-Reaktionsfähigkeitsparameter-Bestimmungssignalen (baseline_up, baseline_down) und den zweiten Fluid-Reaktionsfähigkeitsparameter-Bestimmungssignalen (ripple_up, ripple_down).

2. System (100) nach Anspruch 1, wobei die Bestimmungseinheit (104) verarbeiteter Signale dazu konfiguriert ist, das erste und das zweite verarbeitete Signal (env_up, env_down) durch Anwenden eines Filters auf eine oszillatorische Komponente des Blutpulsationssignals zu erhalten, wobei die Fluid-Reaktionsfähigkeitsparameter-Bestimmungseinheit (105) dazu konfiguriert ist, ein gefiltertes Fluid-Reaktionsfähigkeitsparameter-Bestimmungssignal (env_ripple_up, env_ripple_sum) basierend auf den zweiten Fluid-Reaktionsfähigkeitsparameter-Bestimmungssignalen (ripple_up, ripple_down) und einer weiteren Anwendung eines Filters zu bestimmen, und den Fluid-Reaktionsfähigkeitsparameter (SPV, PPV) basierend auf den ersten Fluid-Reaktionsfähigkeitsparameter-Bestimmungssignalen (baseline_up, baseline_down) und dem gefilterten Fluid-Reaktionsfähigkeitsparameter-Bestimmungssignal (env_ripple_up, env_ripple_sum) zu bestimmen.

3. System (100) nach Anspruch 2, wobei die Fluid-Reaktionsfähigkeitsparameter-Bestimmungseinheit (105) dazu konfiguriert ist, den Fluid-Reaktionsfähigkeitsparameter (SPV, PPV) basierend auf Kennwerten der ersten Fluid-Reaktionsfähigkeitsparameter-Bestimmungssignale (baseline_up, baseline_down) und dem gefilterten Fluid-Reaktionsfähigkeitsparameter-Bestimmungssignal (env_ripple_up, env_ripple_sum) zu bestimmen.

4. System (100) nach einem der vorstehenden Ansprüche, wobei die Bestimmungseinheit (104) verarbeiteter Signale dazu konfiguriert ist, ein erstes verarbeitetes Signal (env_up) zu bestimmen, das durch Anwenden eines Filters auf eine oszillatorische Komponente des Blutpulsationssignals erlangt wird, und eines zweiten verarbeiteten Signasl (env_down), das durch Umkehren der oszillatorischen Komponente und Anwenden des Filters auf die umgekehrte oszillatorische Komponente erlangt wird, und wobei die Fluid-Reaktionsfähigkeitsparameter-Bestimmungseinheit (105) dazu konfiguriert ist, den Fluid-Reaktionsfähigkeitsparameter (SPV) zu bestimmen durch:

   a') Identifizieren eines ersten Fluid-Reaktionsfähigkeitsparameter-Bestimmungssignals für jedes des ersten verarbeiteten Signals (env_up) und des zweiten verarbeiteten Signals (env_down), und Kombinieren der ersten Fluid-Reaktionsfähigkeitsparameter-Bestimmungssignale (baseline_up, baseline_down) zu einem kombinierten ersten Fluid-Reaktionsfähigkeitsparameter-Bestimmungssignal (baseline_sum), Identifizieren eines zweiten Fluid-Reaktionsfähigkeitsparameter-Bestimmungssignals (ripple_up) für das erste verarbeitete Signal (env_up), Bestimmen eines gefilterten Fluid-Reaktionsfähigkeitsparameter-Bestimmungssignals (env_ripple_up) durch Anwenden eines Filters auf das zweite Fluid-Reaktionsfähigkeitsparameter-Bestimmungssignal (ripple_up), das für das erste verarbeitete Signal (env_up) identifiziert wurde, und

b') Bestimmen des Fluid-Reaktionsfähigkeitsparameters (SPV) basierend auf dem kombinierten ersten Fluid-Reaktionsfähigkeitsparameter-Bestimmungssignal (baseline_sum) und dem gefilterten Fluid-Reaktionsfähigkeitsparameter-Bestimmungssignal (env_ripple_up).

5. System (100) nach einem der vorstehenden Ansprüche, wobei das System weiter Folgendes umfasst:

   - eine Blutdruckkennwert-Bereitstellungseinheit zum Bereitstellen eines Blutdruckkennwerts (SAP, DAP),

   wobei die Fluid-Reaktionsfähigkeitsparameter-Bestimmungseinheit (105) dazu konfiguriert ist, den Fluid-Reaktionsfähigkeitsparameter (SPV) weiter basierend auf dem Blutdruckkennwert (SAP, DAP) zu bestimmen.

6. System (100) nach einem der vorstehenden Ansprüche, wobei die Bestimmungseinheit (104) verarbeiteter Signale dazu konfiguriert ist, ein erstes verarbeitetes Signal (env_up) zu bestimmen, das durch Anwenden eines Filters auf eine oszillatorische Komponente des Blutpulsationssignals erhalten wird, und ein zweites verarbeitetes Signal (env_down), das durch Umkehren der oszillatorischen Komponente und Anwenden des Filters auf die umgekehrte oszillatorische Komponente erhalten wird, und wobei die Fluid-Reaktionsfähigkeitsparameter-Bestimmungseinheit (105) dazu konfiguriert ist, den Fluid-Reaktionsfähigkeitsparameter (PPV) zu bestimmen durch:

   a") Identifizieren eines ersten Fluid-Reaktionsfähigkeitsparameter-Bestimmungssignals für jedes der verarbeiteten Signale (env_up, env_down), und Kombinieren dieser zu einem kombinierten ersten Fluid-Reaktionsfähigkeitsparameter-Bestimmungssignal (baseline_sum), Identifizieren eines zweiten Fluid-Reaktionsfähigkeitsparameter-Bestimmungssignals für jedes der verarbeiteten Signale (env_up, env_down) und Kombinieren dieser zu einem kombinierten zweiten Fluid-Reaktionsfähigkeitsparameter-Bestimmungssignal (ripple_sum), und Bestimmen eines gefilterten kombinierten zweiten Fluid-Reaktionsfähigkeitsparameter-Bestimmungssignals (env_ripple_sum) durch weiteres Anwenden eines Filters auf das kombinierte zweite Fluid-Reaktionsfähigkeitsparameter-Bestimmungssignal (ripple_sum), und

   b") Bestimmen des Fluid-Reaktionsfähigkeitsparameters (PPV) basierend auf dem kombinierten ersten Fluid-Reaktionsfähigkeitsparameter-Bestimmungssignals (baseline_sum) und dem gefilterten kombinierten zweiten Fluid-Reaktionsfähigkeitsparameter-Bestimmungssignal (env_ripple_sum).

7. System (100) nach einem der vorstehenden Ansprüche, wobei die Bestimmung des ersten und des zweiten verarbeiteten Signals (env_up, env_down) basierend auf dem Blutpulsationssignals durch die Verarbeitungssignal-Bestimmungseinheit mit einer Regularisierung von erfassten Blutpulsen (PP_ect) in dem Blutpulsationssignal, die auf ektopische Schläge zurückzuführen sind, einhergeht.

8. System (1500) zum Bestimmen eines hämodynamischen Parameters (SV), der mit einem Fluid-Reaktionsfähigkeitsparameter (SPV, PPV) korreliert, wobei das System Folgendes umfasst:

   - das System (100) nach einem von Anspruch 1 bis 7,
   - eine Atemfrequenz-Bereitstellungseinheit (501), die dazu konfiguriert ist, die Atemfrequenz bereitzustellen, auf deren Basis der Fluid-Reaktionsfähigkeitsparameter (SPV, PPV) bestimmt wurde,
   - eine Herzfrequenz-Bereitstellungseinheit (502), die dazu konfiguriert ist, die Herzfrequenz bereitzustellen, auf deren Basis der Fluid-Reaktionsfähigkeitsparameter (SPV, PPV) bestimmt wurde,
   - eine Verarbeitungssignal-Bereitstellungseinheit (503), die dazu konfiguriert ist, das erste und das zweite verarbeitete Signal (env_up, env_down) bereitzustellen, auf deren Basis der Fluid-Reaktionsfähigkeitsparameter (SPV, PPV) bestimmt wurde,
   - eine zweite Bereitstellungseinheit (504) für Fluid-Reaktionsfähigkeitsparameter-Bestimmungssignale, die dazu konfiguriert ist, die zweiten Fluid-Reaktionsfähigkeitsparameter-Bestimmungssignale (ripple_up, ripple_down) des ersten und des zweiten verarbeiteten Signals (env_up, env_down) bereitzustellen, und
   - eine Zuverlässigkeitsbestimmungseinheit (505), die dazu konfiguriert ist, die Zuverlässigkeit (QI)

   des bestimmten Fluid-Reaktionsfähigkeitsparameters (SPV, PPV) zu bestimmen durch:

a) Identifizieren eines ersten Zuverlässigkeitsbestimmungssignals (ripple_up2) basierend auf dem ersten verarbeiteten Signals (env_up), und Identifizieren eines zweiten Zuverlässigkeitsbestimmungssignals (ripple_down2) basierend auf dem zweiten verarbeiteten Signals (env_down), wobei das erste und das zweite Zuverlässigkeitsbestimmungssignal (ripple_up2, ripple_down2) dem ersten und dem zweiten verarbeiteten Signal (env_up, env_down) in einem Spektralbereich zwischen der Atemfrequenz und der Herzfrequenz entsprechen, und

b) Bestimmen der Zuverlässigkeit (QI) des bestimmten Fluid-Reaktionsfähigkeitsparameters (SPV, PPV) basierend auf den zweiten Fluid-Reaktionsfähigkeitsparameter-Bestimmungssignalen (ripple_up, ripple_down) und dem ersten und dem zweiten Zuverlässigkeitsbestimmungssignal (ripple_up2, ripple_down2),

- eine erste Modellbereitstellungseinheit (1510), die dazu konfiguriert ist, ein erstes Modell des hämodynamischen Parameters bereitzustellen, das von dem Fluid-Reaktionsfähigkeitsparameter (SPV, PPV) abhängt, der von dem System (100) nach einem des Anspruchs 1 bis 7 bestimmt wurde,

- eine zweite Modellbereitstellungseinheit (1520), die dazu konfiguriert ist, ein zweites Modell des hämodynamischen Parameters bereitzustellen, das nicht von dem Fluid-Reaktionsfähigkeitsparameter (SPV, PPV) abhängt, und

- eine Kombinationsmodell-Bestimmungseinheit (1530), die dazu konfiguriert ist, ein kombiniertes Modell basierend auf dem ersten Modell, dem zweiten Modell und der Zuverlässigkeit (QI) des Fluid-Reaktionsfähigkeitsparameters (SPV, PPV) zu bestimmen, und

- eine Hämodynamikparameter-Bestimmungseinheit (1540), die dazu konfiguriert ist, den hämodynamischen Parameter durch Anwenden des kombinierten Modells zu bestimmen.

9. Computerimplementiertes Verfahren (200) zum Bestimmen eines Fluid-Reaktionsfähigkeitsparameters für einen Patienten, wobei der bestimmte Fluid-Reaktionsfähigkeitsparameter eine systolische Druckvariation (SPV) oder eine Pulsdruckvariation (PPV) ist, wobei das Verfahren (200) Folgendes umfasst:

- Bereitstellen (201) einer Atemfrequenz,
- Bereitstellen (202) einer Herzfrequenz,
- Bereitstellen (203) eines Blutpulsationssignals, wobei das Blutpulsationssignal ein gemessenes Signal ist, das eine Reihe von Blutpulsen des Patienten angibt,
- Bestimmen (204) eines ersten verarbeiteten Signals (env_up) basierend auf dem Blutpulsationssignal, wobei das erste verarbeitete Signal eine obere Hüllkurve des Blutpulsationssignals angibt, und eines zweiten verarbeiteten Signals (env_down) basierend auf dem Blutpulsationssignal, wobei das zweite verarbeitete Signal eine untere Hüllkurve des Blutpulsationssignals angibt, und **dadurch gekennzeichnet, dass** das Verfahren weiter Folgendes umfasst:

- Bestimmen (205) des Fluid-Reaktionsfähigkeitsparameters (SPV, PPV) durch:

a) Identifizieren von ersten Fluid-Reaktionsfähigkeitsparameter-Bestimmungssignalen (baseline_up, baseline_down) basierend auf dem ersten und dem zweiten verarbeiteten Signal (env_up, env_down), wobei die ersten Fluid-Reaktionsfähigkeitsparameter-Bestimmungssignale dem ersten und dem zweiten verarbeiteten Signal (env_up, env_down) in einem Spektralbereich bis zu der Atemfrequenz entsprechen, und Identifizieren von zweiten Fluid-Reaktionsfähigkeitsparameter-Bestimmungssignalen (ripple_up, ripple_down) basierend auf dem ersten und dem zweiten verarbeiteten Signal (env_up, env_down), wobei die zweiten Fluid-Reaktionsfähigkeitsparameter-Bestimmungssignale (ripple_up, ripple_down) dem ersten und dem zweiten verarbeiteten Signal (env_up, env_down) bei der Atemfrequenz und allen ihrer Harmonischen bis zu der Herzfrequenz entsprechen, und

b) Bestimmen des Fluid-Reaktionsfähigkeitsparameters (SPV, PPV) basierend auf den ersten Fluid-Reaktionsfähigkeitsparameter-Bestimmungssignalen (baseline_up, baseline_down) und den zweiten Fluid-Reaktionsfähigkeitsparameter-Bestimmungssignalen (ripple_up, ripple_down).

10. Computerimplementiertes Verfahren (2600) zum Bestimmen eines hämodynamischen Parameters (SV), der mit einem Fluid-Reaktionsfähigkeitsparameter (SPV, PPV) korreliert, wobei das Verfahren (2600) Folgendes umfasst:

- Bestimmen eines Fluid-Reaktionsfähigkeitsparameters (SPV, PPV) für einen Patienten durch das Verfahren (200) nach Anspruch 9,
- Bestimmen einer Zuverlässigkeit (QI) des bestimmten Fluid-Reaktionsfähigkeitsparameters (SPV, PPV) durch:

- Bereitstellen (601) der Atemfrequenz, auf deren Basis der Fluid-Reaktionsfähigkeitsparameter (SPV, PPV) bestimmt wurde,
- Bereitstellen (602) der Herzfrequenz, auf deren Basis der Fluid-Reaktionsfähigkeitsparameter (SPV, PPV) bestimmt wurde,
- Bereitstellen (603) des ersten und des zweiten verarbeiteten Signals (env_up, env_down), auf deren Basis der Fluid-Reaktionsfähigkeitsparameter (SPV, PPV) bestimmt wurde,
- Bereitstellen (604) der zweiten Fluid-Reaktionsfähigkeitsparameter-Bestimmungssignale (ripple_up, ripple_down) des ersten und des zweiten verarbeiteten Signals (env_up, env_down), und
- Bestimmen (605) der Zuverlässigkeit (QI) des bestimmten Fluid-Reaktionsfähigkeitsparameters (SPV, PPV) durch:

a) Identifizieren eines ersten Zuverlässigkeitsbestimmungssignals (ripple_up2) basierend auf dem ersten verarbeiteten Signal (env_up), und Identifizieren eines zweiten Zuverlässigkeitsbestimmungssignals (ripple_down2) basierend auf dem zweiten verarbeiteten Signal (env_down), wobei das erste und das zweite Zuverlässigkeitsbestimmungssignal dem ersten und dem zweiten verarbeiteten Signal (env_up, env_down) in einem Spektralbereich zwischen der Atemfrequenz und der Herzfrequenz entsprechen, und
b) Bestimmen der Zuverlässigkeit (QI) des bestimmten Fluid-Reaktionsfähigkeitsparameters (SPV, PPV) basierend auf den zweiten Fluid-Reaktionsfähigkeitsparameter-Bestimmungssignalen (ripple_up, ripple_down) und dem ersten und dem zweiten Zuverlässigkeitsbestimmungssignal (ripple_up2, ripple_down2),

wobei das Verfahren (2600) zum Bestimmen eines hämodynamischen Parameters (SV), der mit einem Fluid-Reaktionsfähigkeitsparameter (SPV, PPV) korreliert, weiter Folgendes umfasst:

- Bereitstellen (2610) eines ersten Modells des hämodynamischen Parameters, das von dem bestimmten Fluid-Reaktionsfähigkeitsparameter (SPV, PPV) abhängt,
- Bereitstellen (2620) eines zweiten Modells des hämodynamischen Parameters, das nicht von dem Fluid-Reaktionsfähigkeitsparameter (SPV, PPV) abhängt,
- Bestimmen (2630) eines kombinierten Modells basierend auf dem ersten Modell, dem zweiten Modell und der bestimmten Zuverlässigkeit (QI) des bestimmten Fluid-Reaktionsfähigkeitsparameters (SPV, PPV), und
- Bestimmen (2640) des hämodynamischen Parameters durch Anwenden des kombinierten Modells.

11. Computerprogramm zum Bestimmen eines Fluid-Reaktionsfähigkeitsparameters (SPV, PPV) für einen Patienten, wobei das Programm Anweisungen umfasst, die das System nach den Ansprüchen 1-7 veranlassen, die Schritte des Verfahrens (200) nach Anspruch 9 durchzuführen, wenn das Programm von dem System ausgeführt wird.

12. Computerprogramm zum Bestimmen eines hämodynamischen Parameters (SV), der mit einem Fluid-Reaktionsfähigkeitsparameter (SPV, PPV) korreliert, der durch das Computerprogramm nach Anspruch 11 bestimmt wurde, wobei das Programm Anweisungen umfasst, die das System nach Anspruch 8 veranlassen, die Schritte des Verfahrens (2600) nach Anspruch 10 auszuführen, wenn das Programm von dem System ausgeführt wird.

**Revendications**

1. Système (100) permettant de déterminer un paramètre de sensibilité fluidique pour un patient, dans lequel ledit paramètre de sensibilité fluidique est une variation de pression systolique (SPV) ou une variation de pression différentielle (PPV), dans lequel le système comprend :

- une unité de fourniture de fréquence respiratoire (101) configurée pour fournir une fréquence respiratoire,
- une unité de fourniture de fréquence cardiaque (102) configurée pour fournir une fréquence cardiaque,
- une unité de fourniture de signal de pulsations sanguines (103) configurée pour fournir un signal de pulsations sanguines, dans lequel le signal de pulsations sanguines est un signal mesuré indicatif d'une série de pulsations sanguines du patient,
- une unité de détermination de signal traité (104) configurée pour déterminer un premier signal traité (env_up) sur la base du signal de pulsations sanguines, dans lequel le premier signal traité est indicatif d'une enveloppe supérieure du signal de pulsations sanguines, et un second signal traité (env_down) sur la base du signal de pulsations sanguines, dans lequel le second signal traité indique une enveloppe inférieure du signal de pulsations sanguines, et

- **caractérisé en ce que** le système comprend en outre une unité de détermination de paramètre de sensibilité fluidique (105) configurée pour déterminer le paramètre de sensibilité fluidique (SPV, PPV) par :

a) l'identification de premiers signaux de détermination de paramètre de sensibilité fluidique (baseline_up, baseline_down) sur la base des premier et second signaux traités (env_up, env_down), dans lequel les premiers signaux de détermination de paramètre de sensibilité fluidique correspondent aux premier et second signaux traités (env_up, env_down) dans une gamme spectrale allant jusqu'à la fréquence respiratoire, et l'identification de seconds signaux de détermination de paramètres de réactivité fluidique (ripple_up, ripple_down) sur la base des premier et second signaux traités (env_up, env_down), dans lequel les seconds signaux de détermination de paramètre de sensibilité fluidique (ripple_up, ripple_down) correspondent aux premier et second signaux traités (env_up, env_down) à la fréquence respiratoire et à l'une de ses harmoniques jusqu'à la fréquence cardiaque, et

b) la détermination de paramètre de sensibilité fluidique (SPV, PPV) sur la base des premiers signaux de détermination de paramètre de sensibilité fluidique (baseline_up, baseline_down) et des seconds signaux de détermination de paramètre de sensibilité fluidique (ripple_up, ripple_down).

2. Système (100) selon la revendication 1, dans lequel l'unité de détermination de signal traité (104) est configurée pour obtenir les premier et second signaux traités (env_up, env_down) par l'application d'un filtre à une composante oscillatoire du signal de pulsations sanguines, dans lequel l'unité de détermination de paramètre de sensibilité fluidique (105) est configurée pour déterminer un signal de détermination de paramètre de sensibilité fluidique filtré (env_ripple_up, env_ripple_sum) sur la base des seconds signaux de détermination de paramètre de sensibilité fluidique (ripple_up, ripple_down) et une application supplémentaire d'un filtre, et pour déterminer le paramètre de sensibilité fluidique (SPV, PPV) sur la base des premiers signaux de détermination de paramètre de sensibilité fluidique (baseline_up, baseline_down) et du signal de détermination de paramètre de sensibilité fluidique filtré (env_ripple_up, env_ripple_sum).

3. Système (100) selon la revendication 2, dans lequel l'unité de détermination de paramètre de sensibilité fluidique (105) est configurée pour déterminer le paramètre de sensibilité fluidique (SPV, PPV) sur la base de valeurs caractéristiques des premiers signaux de détermination de paramètre de sensibilité fluidique (baseline_up, baseline_down) et du signal de détermination de paramètre de sensibilité fluidique filtré (env_ripple_up, env_ripple_sum).

4. Système (100) selon l'une quelconque des revendications précédentes, dans lequel l'unité de détermination de signal traité (104) est configurée pour déterminer un premier signal traité (env_up) obtenu par l'application d'un filtre à une composante oscillatoire du signal de pulsations sanguines et un second signal traité (env_down) obtenu par l'inversion de la composante oscillatoire et par l'application du filtre à la composante oscillatoire inversée, et dans lequel l'unité de détermination de paramètre de sensibilité fluidique (105) est configurée pour déterminer le paramètre de sensibilité fluidique (SPV) par :

a') l'identification d'un premier signal de détermination de paramètre de sensibilité fluidique pour chacun du premier signal traité (env_up) et du second signal traité (env_down) et la combinaison des premiers signaux de détermination de paramètre de sensibilité fluidique (baseline_up, baseline_down) à un premier signal combiné de détermination de paramètre de sensibilité fluidique (baseline_sum), l'identification d'un second signal de détermination de paramètre de sensibilité fluidique (ripple_up) pour le premier signal traité (env_up), la détermination d'un signal de détermination de paramètre de sensibilité fluidique filtré (env_ripple_up) par l'application d'un filtre au second signal de détermination de paramètre de sensibilité fluidique (ripple_up) identifié pour le premier signal traité (env_up), et

b') la détermination du paramètre de sensibilité fluidique (SPV) sur la base du premier signal combiné de détermination de paramètre de sensibilité fluidique (baseline_sum) et du signal de détermination de paramètre de sensibilité fluidique filtré (env_ripple_up).

5. Système (100) selon l'une quelconque des revendications précédentes, dans lequel le système comprend en outre :

- une unité de fourniture de caractéristique de pression artérielle pour fournir une caractéristique de pression artérielle (SAP, DAP),

dans lequel l'unité de détermination de paramètre de sensibilité fluidique (105) est configurée pour déterminer le paramètre de sensibilité fluidique (SPV) sur la base en outre de la caractéristique de pression artérielle (SAP, DAP).

6. Système (100) selon l'une quelconque des revendications précédentes, dans lequel l'unité de détermination de signal traité (104) est configurée pour déterminer un premier signal traité (env_up) obtenu par l'application d'un filtre à une composante oscillatoire du signal de pulsations sanguines et un second signal traité (env_down) obtenu par l'inversion de la composante oscillatoire et par l'application du filtre à la composante oscillatoire inversée, et dans lequel l'unité de détermination de paramètre de sensibilité fluidique (105) est configurée pour déterminer le paramètre de sensibilité fluidique (PPV) par :

a'') l'identification d'un premier signal de détermination de paramètre de sensibilité fluidique pour chacun des signaux traités (env_up, env_down) et la combinaison de ceux-ci en un premier signal combiné de détermination de paramètre de sensibilité fluidique (baseline_sum), l'identification d'un second signal de détermination de paramètre de sensibilité fluidique pour chacun des signaux traités (env_up, env_down) et la combinaison de ceux-ci en un second signal combiné de détermination de paramètre de sensibilité fluidique (ripple_sum), et la détermination d'un second signal combiné filtré de détermination de paramètre de sensibilité fluidique (env_ripple_sum) par l'application en outre d'un filtre au second signal combiné de détermination de paramètre de sensibilité fluidique (ripple_sum), et
b'') la détermination du paramètre de sensibilité fluidique (PPV) sur la base du premier signal combiné de détermination de paramètre de sensibilité fluidique (baseline_sum) et du second signal combiné filtré de détermination de paramètre de sensibilité fluidique (env_ripple_sum).

7. Système (100) selon l'une quelconque des revendications précédentes, dans lequel la détermination des premier et second signaux traités (env_up, env_down) sur la base du signal de pulsations sanguines par l'unité de détermination de signal traité implique une régularisation des pulsations sanguines détectées (PP_ect) dans le signal de pulsations sanguines qui sont dues à des battements ectopiques.

8. Système (1500) permettant de déterminer un paramètre hémodynamique (SV) corrélé à un paramètre de sensibilité fluidique (SPV, PPV), dans lequel le système comprend :

- le système (100) selon l'une quelconque des revendications 1 à 7,
- une unité de fourniture de fréquence respiratoire (501) configurée pour fournir la fréquence respiratoire sur la base de laquelle le paramètre de sensibilité fluidique (SPV, PPV) a été déterminé,
- une unité de fourniture de fréquence cardiaque (502) configurée pour fournir la fréquence cardiaque sur la base de laquelle le paramètre de sensibilité fluidique (SPV, PPV) a été déterminé,
- une unité de fourniture de signal traité (503) configurée pour fournir les premier et second signaux traités (env_up, env_down) sur la base desquels le paramètre de sensibilité fluidique (SPV, PPV) a été déterminé,
- une seconde unité de fourniture de signaux de détermination de paramètre de sensibilité fluidique (504) configurée pour fournir les seconds signaux de détermination de paramètre de sensibilité fluidique (ripple_up, ripple_down) des premier et second signaux traités (env_up, env_down), et
- une unité de détermination de fiabilité (505) configurée pour déterminer la fiabilité (QI)

du paramètre de sensibilité fluidique déterminé (SPV, PPV) par :

a) l'identification d'un premier signal de détermination de fiabilité (ripple_up2) sur la base du premier signal traité (env_up), et l'identification d'un second signal de détermination de fiabilité (ripple_down2) sur la base du second signal traité (env_down), dans lequel les premier et second signaux de détermination de fiabilité (ripple_up2, ripple_down2) correspondent aux premier et second signaux traités (env_up, env_down) dans une gamme spectrale comprise entre la fréquence respiratoire et la fréquence cardiaque, et
b) la détermination de la fiabilité (QI) du paramètre de sensibilité fluidique déterminé (SPV, PPV) sur la base des seconds signaux de détermination de paramètre de sensibilité fluidique (ripple_up, ripple_down) et des premier et second signaux de détermination de fiabilité (ripple_up2, ripple_down2).

- une première unité de fourniture de modèle (1510) configurée pour fournir un premier modèle du paramètre hémodynamique qui dépend du paramètre de sensibilité fluidique (SPV, PPV) déterminé par le système (100) selon l'une quelconque des revendications 1 à 7,
- une seconde unité de fourniture de modèle (1520) configurée pour fournir un second modèle du paramètre hémodynamique qui ne dépend pas du paramètre de sensibilité fluidique (SPV, PPV), et
- une unité de détermination de modèle combiné (1530) configurée pour déterminer un modèle combiné sur la base du premier modèle, du second modèle et de la fiabilité (QI) du paramètre de sensibilité fluidique (SPV, PPV), et
- une unité de détermination de paramètre hémodynamique (1540) configurée pour déterminer le paramètre hémodynamique par l'application du modèle combiné.

9. Procédé mis en œuvre par ordinateur (200) pour déterminer un paramètre de sensibilité fluidique pour un patient, dans lequel le paramètre de sensibilité fluidique déterminé est une variation de pression systolique (SPV) ou une variation de pression différentielle (PPV), dans lequel le procédé (200) comprend :

- la fourniture (201) d'une fréquence respiratoire,
- la fourniture (202) d'une fréquence cardiaque,
- la fourniture (203) d'un signal de pulsations sanguines, dans lequel le signal de pulsations sanguines est un signal mesuré indicatif d'une série de pulsations sanguines du patient,
- la détermination (204) d'un premier signal traité (env_up) sur la base du signal de pulsations sanguines, dans lequel le premier signal traité est indicatif d'une enveloppe supérieure du signal de pulsations sanguines, et d'un second signal traité (env_down) sur la base du signal de pulsations sanguines, dans lequel le second signal traité est indicatif d'une enveloppe inférieure du signal de pulsations sanguines, et **caractérisé en ce que** le procédé comprend en outre :

- la détermination (205) du paramètre de sensibilité fluidique (SPV, PPV) par :

a) l'identification de premiers signaux de détermination de paramètre de sensibilité fluidique (baseline_up, baseline_down) sur la base des premier et second signaux traités (env_up, env_down), dans lequel les premiers signaux de détermination de paramètre de sensibilité fluidique correspondent aux premier et second signaux traités (env_up, env_down) dans une gamme spectrale allant jusqu'à la fréquence respiratoire, et l'identification de seconds signaux de détermination de paramètres de réactivité fluidique (ripple_up, ripple_down) sur la base des premier et second signaux traités (env_up, env_down), dans lequel les seconds signaux de détermination de paramètre de sensibilité fluidique (ripple_up, ripple_down) correspondent aux premier et second signaux traités (env_up, env_down) à la fréquence respiratoire et à l'une de ses harmoniques jusqu'à la fréquence cardiaque, et
b) la détermination de paramètre de sensibilité fluidique (SPV, PPV) sur la base des premiers signaux de détermination de paramètre de sensibilité fluidique (baseline_up, baseline_down) et des seconds signaux de détermination de paramètre de sensibilité fluidique (ripple_up, ripple_down).

10. Procédé mis en œuvre par ordinateur (2600) pour déterminer un paramètre hémodynamique (SV) corrélé à un paramètre de sensibilité fluidique (SPV, PPV), dans lequel le procédé (2600) comprend :

- la détermination un paramètre de sensibilité fluidique (SPV, PPV) pour un patient par le procédé (200) selon la revendication 9,
- la détermination d'une fiabilité (QI) du paramètre de sensibilité fluidique déterminé (SPV, PPV) par :

- la fourniture (601) de la fréquence respiratoire sur la base de laquelle le paramètre de sensibilité fluidique (SPV, PPV) a été déterminé,
- la fourniture (602) de la fréquence cardiaque sur la base de laquelle le paramètre de sensibilité fluidique (SPV, PPV) a été déterminé,
- la fourniture (603) des premier et second signaux traités (env_up, env_down) sur la base desquels le paramètre de sensibilité fluidique (SPV, PPV) a été déterminé,
- la fourniture (604) des seconds signaux de détermination de paramètre de sensibilité fluidique (ripple_up, ripple_down) des premier et second signaux traités (env_up, env_down), et
- la détermination (605) de la fiabilité (QI) du paramètre de sensibilité fluidique déterminé (SPV, PPV) par :

a) l'identification d'un premier signal de détermination de fiabilité (ripple_up2) sur la base du premier signal traité (env_up), et l'identification d'un second signal de détermination de fiabilité (ripple_down2) sur la base du second signal traité (env_down), dans lequel les premier et second signaux de détermination de fiabilité correspondent aux premier et second signaux traités (env_up, env_down) dans une gamme spectrale comprise entre la fréquence respiratoire et la fréquence cardiaque, et
b) la détermination de la fiabilité (QI) du paramètre de sensibilité fluidique déterminé (SPV, PPV) sur la base des seconds signaux de détermination de paramètre de sensibilité fluidique (ripple_up, ripple_down) et des premier et second signaux de détermination de fiabilité (ripple_up2, ripple_down2),

le procédé (2600) de détermination d'un paramètre hémodynamique (SV) corrélé à un paramètre de sensibilité fluidique (SPV, PPV) comprenant en outre :

- la fourniture (2610) d'un premier modèle du paramètre hémodynamique qui dépend du paramètre de sensibilité fluidique déterminé (SPV, PPV),
- la fourniture (2620) d'un second modèle du paramètre hémodynamique qui ne dépend pas du paramètre de sensibilité fluidique (SPV, PPV),
- la détermination (2630) d'un modèle combiné sur la base du premier modèle, du second modèle et de la fiabilité déterminée (QI) du paramètre de sensibilité fluidique déterminé (SPV, PPV), et
- la détermination (2640) du paramètre hémodynamique par l'application du modèle combiné.

11. Programme informatique permettant de déterminer un paramètre de sensibilité fluidique (SPV, PPV) pour un patient, dans lequel le programme comprend des instructions amenant le système selon les revendications 1-7 à réaliser les étapes du procédé (200) selon la revendication 9, si le programme est exécuté par le système.

12. Programme informatique permettant de déterminer un paramètre hémodynamique (SV), corrélé à un paramètre de sensibilité fluidique (SPV, PPV) déterminé par le programme informatique selon la revendication 11, dans lequel le programme comprend des instructions amenant le système selon la revendication 8 à réaliser les étapes du procédé (2600) selon la revendication 10, si le programme est exécuté par le système.

100

101

102

103

104

105

# FIG. 1

FIG. 2

FIG. 3

FIG. 4A

FIG. 4B

500

501

502

503

504

505

# FIG. 5

FIG. 6

FIG. 7A

FIG. 7B

FIG. 8A

FIG. 8B

FIG. 8C

FIG. 8D

FIG. 8E

1500

100

500

1510

1520

1530

1540

FIG. 9

200

201

202

203

204

205

# FIG. 10

600

|     | |     |
|-----|-|-----|
| 601 | | |

↓

| 602 |

↓

| 603 |

↓

| 604 |

↓

| 605 |

# FIG. 11

2600

FIG. 12

FIG. 13

FIG. 14A

FIG. 14B

EP 4 539 733 B1

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2011077532 A1
- US 2020260969 A1
- US 2008033306 A1
- EP 3430992 A1
- WO 2020148137 A1
- WO 2019211210 A1

### Non-patent literature cited in the description

- **M. CANNESSON et al.** Predicting fluid responsiveness with stroke volume variation despite multiple extrasystoles. *Critical Care Medicine*, 2012, vol. 40, 193-198
- **T. CHEN et al.** The Effect of Signal Quality on Six Cardiac Output Estimators. *36th Annual Computers in Cardiology Conference*, 2009, 197-200